(19) Europäisches Patentamt
European Patent Office
Office européen des brevets

(11) EP 2 745 850 B1

(12) # EUROPEAN PATENT SPECIFICATION

(45) Date of publication and mention of the grant of the patent:
**16.05.2018 Bulletin 2018/20**

(21) Application number: **12832548.7**

(22) Date of filing: **12.09.2012**

(51) Int Cl.:
***A61K 51/00*** (2006.01)
***A61B 5/055*** (2006.01)
***A61B 8/08*** (2006.01)
***A61N 5/10*** (2006.01)
***G01T 1/161*** (2006.01)
***A61B 6/12*** (2006.01)
***A61B 18/14*** (2006.01)
***A61K 51/06*** (2006.01)
***A61B 5/06*** (2006.01)
***A61K 51/12*** (2006.01)
***A61B 1/00*** (2006.01)
***A61B 6/03*** (2006.01)
***A61K 31/765*** (2006.01)
***A61P 35/00*** (2006.01)
***A61K 49/00*** (2006.01)
***A61B 6/00*** (2006.01)
***A61B 18/18*** (2006.01)
***A61B 5/08*** (2006.01)
***A61B 18/02*** (2006.01)

(86) International application number:
**PCT/JP2012/073346**

(87) International publication number:
**WO 2013/039111 (21.03.2013 Gazette 2013/12)**

(54) **NANO-PARTICLES FOR INTERNAL RADIATION THERAPY OF INVOLVED AREA, AND THERAPY SYSTEM**

NANOPARTIKEL ZUR INTERNEN STRAHLENTHERAPIE EINES BETROFFENEN BEREICHS UND THERAPIESYSTEM

NANOPARTICULES POUR RADIOTHÉRAPIE INTERNE D'UNE ZONE CONCERNÉE ET SYSTÈME DE THÉRAPIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.09.2011 JP 2011203725**

(43) Date of publication of application:
**25.06.2014 Bulletin 2014/26**

(73) Proprietors:
- **Shimadzu Corporation**
  **Kyoto 604-8511 (JP)**
- **Kyoto University**
  **Kyoto-shi, Kyoto 606-8501 (JP)**

(72) Inventors:
- **TAKEUCHI, Eri**
  **Kyoto-shi**
  **Kyoto 604-8511 (JP)**
- **HARA, Isao**
  **Kyoto-shi**
  **Kyoto 604-8511 (JP)**
- **YAMAHARA, Ryo**
  **Kyoto-shi**
  **Kyoto 604-8511 (JP)**
- **OZEKI, Eiichi**
  **Kyoto-shi**
  **Kyoto 604-8511 (JP)**
- **KIMURA, Shunsaku**
  **Kyoto-shi**
  **Kyoto 606-8501 (JP)**
- **KURIHARA, Kensuke**
  **Kyoto-shi**
  **Kyoto 606-8501 (JP)**

(74) Representative: **Kilian Kilian & Partner**
**Aidenbachstraße 54**
**81379 München (DE)**

(56) References cited:
**WO-A1-2009/148121**
**WO-A1-2011/099524**
**CA-A1- 2 789 310**
**JP-A- 2008 253 585**
**JP-A- 2009 045 251**
**JP-A- 2011 506 343**
**US-A1- 2011 104 056**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **KENSUKE KURIHARA ET AL.: 'Shinki Nanocarrier, Lactosome o Mochiita Noshuyo Dosho Ishoku Model Mouse ni Okeru Keiko Imaging Oyobi Byori Soshiki tono Hikaku' DRUG DLIVERY SYSTEM vol. 25, no. 3, 2010, page 315, XP008173077**
- **SHUNSAKU KIMURA ET AL.: 'Polydepsipeptide Nanocarrier o Mochiita Kokeigan no Bunshi Imaging to Naishosha Chiryo' THE ANNUAL MEETING OF THE JAPANESE SOCIETY FOR BIOMATERIALS YOKOSHU vol. 33, 21 November 2011, page 207, XP008173363**

## Description

### TECHNICAL FIELD

The present invention relates to a nanoparticle for internal radiation therapy of a lesion site. The present invention also relates to a system for treating a lesion site where many neo vessels are generated, such as a cancer, with a combination of percutaneous local therapy and internal radiation therapy using nanoparticles.

### BACKGROUND ART

As a method for treating malignant tumors such as cancers, internal radiation therapy using a compound containing a β-emitters (e.g., $^{131}I$, $^{90}Y$, $^{177}Lu$) is currently known in addition to chemotherapy, surgical therapy involving the removal of an affected area, radiation therapy involving the exposure of an affected area to radiation, and percutaneous local therapy (i.e., percutaneous local ablative therapy (ablation)).

As described in Non-Patent Document 1 (Abstract of The 50th Annual Scientific Meeting of the Japanese Society of Nuclear Medicine, 2010, p. 316-321), internal radiation therapy with radioactive iodine 131 ($^{131}I$) has been used for 65 years and is now an essential treatment method for thyroid cancer and Graves' disease. Particularly, a method for treating thyroid cancer by destroying only transfer cells having the ability to metabolize iodine is a target medical treatment model.

Internal radiation therapy with $^{131}$IMIBG (Meta-iodobenzylguanitidine) has been used in Europe and the United States since 1984. This method is used for treatment of malignant neuroendocrine tumors such as melanocytoma, paraganglioma, carcinoid, medullary thyroid cancer, and neuroblastoma for the purpose of shrinking tumors and relieving various symptoms, such as hypertension and palpitations, produced when a surgical operation is impossible or clinical various symptoms such as pain caused by bone metastasis.

In recent years, radioimmunotherapy with a $^{90}$Y-labeled anti-CD20 antibody (ibritumomab) has been used for treatment of malignant lymphoma.

Under the circumstances, the number of cases of internal radiation therapy tends to increase, and the number of medical treatment facilities is increasing. In Japan, it has become possible to use up to 500 MBq of $^{131}I$ for outpatient therapy, and support for internal radiation therapy by health-care providers is also expanding. Further, regarding thyroid cancer, an additional fee for radiotherapy patient room management and an increase in fee for radioisotope internal radiation therapy management have been approved since April of 2010.

WO 2009/148121 (Patent Document 1) discloses a nanoparticle (lactosome) comprising an amphiphilic block polymer comprising a hydrophilic block having a sarcosine unit and a hydrophobic block having a lactic acid unit.

### PRIOR ART DOCUMENTS

#### PATENT DOCUMENT

WO 2009/148121

#### NON-PATENT DOCUMENT

Non-Patent Document 1: Abstract of The 50th Annual Scientific Meeting of the Japanese Society of Nuclear Medicine, 2010, p. 316-321

### SUMMARY OF THE INVENTION

#### PROBLEMS TO BE SOLVED BY THE INVENTION

Cancer therapies such as chemotherapy, surgery, local therapy, and radiation therapy are directed to minimize a treatment area as much as possible and reduce the burden on patients. Therefore, local therapy is often used. However, such therapy has a problem that untreated tumor cells remaining around the edge portion of a treated tumor site are likely to cause recurrence or metastasis.

Further, current internal radiation therapy is targeted therapy that can be used only for thyroid and malignant lymphoma, and therefore there is a problem that internal radiation therapy cannot be applied to treatment of other lesions (especially, solid cancers).

Further, radioimmunotherapy uses an antibody, and therefore has the problem of high drug prices.

On the other hand, the present inventors have prepared an $^{131}$I-lactosome that is a lactosome encapsulating

a radioactive $^{131}$I-labeled polylactic acid (lactosome is a particle formed by self-assembly of an amphiphilic substance, as a constitutional element, having a polylactic acid block) . The present inventors have confirmed that tumors can be treated with internal radiation therapy using this $^{131}$I-lactosome as an internal radiation therapeutic agent. However, when a tumor having a large volume is treated with such therapy, the lactosome having a high radiation value needs to be administered. Further, the lactosome has excellent blood retentivity, and therefore adverse effects on normal tissues may be increased by the retention of the administered lactosome having a high radiation value in blood. This may result in radiation side effects such as bone-marrow suppression and body weight loss.

Therefore, an object of the present invention is to provide a therapeutic system that is widely applicable to general solid cancers, can achieve both a reduction in side effects of cancer therapy and suppression of cancer recurrence and metastasis, and requires no expensive drug such as an antibody. Another object of the present invention is to provide a nanoparticle for internal radiation therapy to be used in the therapeutic system.

MEANS FOR SOLVING THE PROBLEMS

The present inventors have found that the above objects can be achieved by providing a therapeutic system with which percutaneous local therapy can be performed in advance, and then internal radiation therapy using a lactosome encapsulating a β-ray emitting nuclide-labeled substance can be performed, which has led to the completion of the present invention.

The present invention includes the followings.

(1) A nanoparticle for internal radiation therapy of a lesion site, comprising:

an amphiphilic block polymer comprising a hydrophilic block having a sarcosine unit and a hydrophobic block having a lactic acid unit; and
a substance labeled with a β-ray emitting nuclide.

(2) The nanoparticle according to the above (1), wherein the β-ray emitting nuclide is selected from the group consisting of iodine-131, yttrium-90, and lutetium-177.
(3) The nanoparticle according to the above (1) or (2), wherein the amphiphilic block polymer comprises a hydrophilic block having 20 or more sarcosine units and a hydrophobic block having 10 or more lactic acid units.
(4) The nanoparticle according to any one of the above (1) to (3), wherein the nanoparticle has a particle size of 10 nm to 200 nm.
(5) The nanoparticle according to any one of the above (1) to (4), wherein the substance labeled with a β-ray emitting nuclide is polylactic acid labeled with a β-ray emitting nuclide.
(6) A system for internal radiation therapy of a lesion site comprising:

a device comprising a means for acquiring image data showing a position of a lesion site, and a means for positioning a needle, which should be punctured into the lesion site, at the lesion site based on the image data; and
a nanoparticle comprising an amphiphilic block polymer comprising a hydrophilic block having a sarcosine unit and a hydrophobic block having a lactic acid unit, and a substance labeled with a β-ray emitting nuclide.

In the above (6), the needle is used for percutaneous local therapy, and the nanoparticle is used as an internal radiation therapeutic agent.
(7) The system according to the above (6), wherein the needle is selected from the group consisting of an injection needle to supply ethanol, an injection needle to supply gas, a radiofrequency electrode needle, and a microwave electrode needle.
In the above (7), the injection needle to supply ethanol is used for percutaneous ethanol injection therapy, the injection needle to supply gas is used for cryotherapy, the radiofrequency electrode needle is used for radiofrequency ablation, and the microwave electrode needle is used for microwave coagulation therapy.
(8) The system according to the above (6) or (7), wherein the β-ray emitting nuclide is selected from the group consisting of iodine-131, yttrium-90, and lutetium-177.
The nanoparticles can be prepared so as to have a radiation value of 10 MBq/kg to 600 MBq/kg as one-time use in the system for a mouse.
(9) The system according to any one of the above (6) to (8), wherein the amphiphilic block polymer comprises a hydrophilic block having 20 or more sarcosine units and a hydrophobic block having 10 or more lactic acid units.
(10) The system according to any one of the above (6) to (9), wherein the nanoparticle has a particle size of 10 nm to 200 nm.

(11) The system according to any one of the above (6) to (10), wherein the substance labeled with a β-ray emitting nuclide is polylactic acid labeled with a β-ray emitting nuclide.

(12) The system according to any one of the above (6) to (11), further comprising a nanoparticle comprising:

an amphiphilic block polymer comprising a hydrophilic block having a sarcosine unit and a hydrophobic block having a lactic acid unit;
and a substance labeled with a γ-ray emitting nuclide.

In the above (12), the substance labeled with a γ-ray emitting nuclide may be polylactic acid labeled with a γ-ray emitting nuclide.

In the above (12), the amphiphilic block polymer may comprise a hydrophilic block having 20 or more sarcosine units and a hydrophobic block having 10 or more lactic acid units.

(13) The system according to the above (12), wherein the γ-ray emitting nuclide is a single photon emitting nuclide.

In the above (13), the nanoparticle containing the substance labeled with a γ-ray emitting nuclide is used as a probe for single photon emission computed tomography.

(14) The system according to the above (12), wherein the γ-ray emitting nuclide is a positron emitting nuclide.

In the above (14), the nanoparticle containing the substance labeled with a γ-ray emitting nuclide is used as a probe for positron emission tomography.

EFFECTS OF THE INVENTION

According to the present invention, it is possible to provide an inexpensive therapeutic system that is widely applicable to general solid cancers, can achieve both a reduction in side effects of cancer therapy and suppression of cancer recurrence and metastasis, and requires no expensive drug such as an antibody; and a nanoparticle for internal radiation therapy.

According to the present invention, after most of a tumor is necrotized with percutaneous local therapy (percutaneous local ablative therapy) and angiogenesis is induced, it is possible to treat remaining tumor tissue untreated with percutaneous local therapy, such as an area around the edge of the tumor, with internal radiation therapy using, as an internal radiation therapeutic agent, an iodine 131 compound-containing lactosome. Such combined therapy makes it possible to maximally utilize the EPR effect that allows the nanoparticle to exhibit its accumulating property. Further, by treating most of a tumor, which should be treated with internal radiation therapy, with percutaneous local therapy prior to internal radiation therapy, it is possible to reduce the radiation value of the iodine 131 compound-containing lactosome as an internal radiation therapeutic agent and therefore to reduce radiation side effects.

BRIEF DESCRIPTION OF THE DRAWINGS

[Fig.1]
Fig. 1 shows HPLC charts during purification of [$^{131}$I] -SIB in Experimental Example 1.
[Fig.2]
Fig. 2 shows an HPLC chart during purification of $^{131}$I-BzPLLA$_{30}$ in Experimental Example 4.
[Fig.3]
Fig. 3 shows fluorescence images showing the results of confirming the accumulation of a lactosome in Experimental Example 8.
[Fig.4]
Fig. 4 shows graphs showing the results of changes in fluorescence intensity analyzed from the fluorescence images in Experimental Example 8.
[Fig.5]
Fig. 5 shows a graph showing the results of measuring the distribution of an $^{131}$I-lactosome in a body in Experimental Example 9.
[Fig.6]
Fig. 6 shows a graph showing the results of an anticancer activity test for an $^{131}$I-lactosome in Experimental Example 10.
[Fig.7]
Fig. 7 shows a graph showing changes in relative tumor volume in an antitumor test using mice in Example 1.

[Fig.8]
Fig. 8 shows a graph showing changes in body weight in the antitumor test using mice in Example 1.
[Fig.9]
Fig. 9 shows a graph showing changes in relative tumor volume in an antitumor test using mice (5 MBq/body) in Reference Example 1.
[Fig.10]
Fig. 10 shows a graph showing changes in body weight in the antitumor test using mice (5 MBq/body) in Reference Example 1.
[Fig.11]
Fig. 11 shows a graph showing changes in relative tumor volume in an antitumor test using mice (5 MBq/body) in Reference Example 2.
[Fig.12]
Fig. 12 shows a graph showing changes in relative tumor volume in an antitumor test using mice (40 MBq/body) in Reference Example 3.
[Fig.13]
Fig. 13 shows a graph showing changes in body weight in the antitumor test using mice (40 MBq/body) in Reference Example 3.

## MODE FOR CARRYING OUT THE INVENTION

### [1. Object to which therapeutic system is applied]

A therapeutic system according to the present invention includes a device for performing percutaneous local therapy and a nanoparticle as an internal radiation therapeutic agent. The nanoparticle as an internal radiation therapeutic agent in the present invention has the property of passing through neo vessels and accumulating in surrounding tissue due to the EPR (enhanced permeability and retention) effect. In the present invention, in-vivo tissue in which many neo vessels are present is collectively referred to as a "vascular lesion". That is, a lesion site as an object to which the therapeutic system according to the present invention is applied is a vascular lesion site.
Since the nanoparticle in the present invention has the property of specifically accumulating in a vascular lesion site, the therapeutic system according to the present invention can be applied to a wide variety of vascular lesions regardless of their type. Specifically, the vascular lesions include tumors, inflammations, arteriosclerosis and the like. The tumors are preferably malignant tumors, that is, cancers. It is to be noted that in the present invention, the cancers are generally solid cancers (i.e., hematopoietic cancers are not included). Examples of the cancers include breast cancer, subcutaneous cancer, liver cancer, lung cancer, pancreas cancer, brain tumor, colorectal cancer and the like.
A living body having a vascular lesion is not particularly limited, and may be a human or a non-human animal. The non-human animal is not particularly limited, and examples thereof include mammals other than humans. More specific examples of the mammals other than humans include primates, rodents (e.g., mice, rats), rabbits, dogs, cats, pigs, bovine, sheep, and horses.

### [2. Device for performing percutaneous local therapy]

The device for performing percutaneous local therapy includes a molecular imaging means and a puncture control means .
The molecular imaging means is, specifically, a means for acquiring image data showing the position of a vascular lesion site. The puncture control means is a means for positioning a needle, which should be punctured into the vascular lesion site, at the vascular lesion site based on the image data.

#### [2-1. Means for acquiring image data showing position of vascular lesion site]

The molecular imaging means, that is, the means for acquiring image date showing the position of a vascular lesion site may be a means capable of specifying a vascular lesion site in a three- or two-dimensional shape, derived from the image data of tissue including the vascular lesion site, by visual observation, automatic image processing, or localization of a contrast medium.
Specific examples of such a means include: shape diagnostic systems such as an ultrasonic diagnostic imaging system, a magnetic resonance imaging (MRI) system, and a computed tomography (CT) system; scintigraphy systems such as a single photon emission computed tomography (SPECT) system and a positron emission tomography (PET) system; and systems for fluorescent imaging.
When the therapeutic system according to the present invention is used, acquisition of image data may be

performed before a vascular lesion site is treated with local therapy or after a vascular lesion site is treated with local therapy and before the vascular lesion site is treated with internal radiation therapy. In either case, treatment of a vascular lesion site is performed after acquisition of image data, and therefore the above-described means is preferably a scintigraphy system.

It is to be noted that when a scintigraphy system is used, a nanoparticle similar to the nanoparticle as an internal radiation therapeutic agent that will be described later is preferably used as a probe for molecular imaging. Specifically, the nanoparticle as a probe for molecular imaging preferably has the same structure as the nanoparticle as an internal radiation therapeutic agent, except that there is a difference in the type of radioisotope used between them. Specifically, such a nanoparticle may contain, as a carrier agent, the same carrier agent as the nanoparticle as an internal radiation therapeutic agent and, as a substance encapsulated in the carrier agent, a substance labeled with a radioisotope for molecular imaging (specifically, a $\gamma$-ray emitting nuclide). Specific examples of the $\gamma$-ray emitting nuclide include a single photon emitting nuclide for a probe for single photon emission computed tomography and a positron emitting nuclide for a probe for positron emission tomography. Specific examples of the single photon emitting nuclide include iodine-123, iodine-125, iodine-131, gallium-67, technetium-99m, indium-111, lutetium-177 and the like. Specific examples of the positron emitting nuclide include iodine-124, carbon-11, nitrogen-13, oxygen-15, fluorine-18, gallium-68, and copper-64.

The probe for molecular imaging uses the same carrier agent as the nanoparticle as an internal radiation therapeutic agent. Therefore, likewise the nanoparticle as an internal radiation therapeutic agent, the probe for molecular imaging has the property of specifically accumulating in a vascular lesion site. It is to be noted that the accumulation of the probe for molecular imaging in a vascular lesion site can be confirmed after 3 hours to 48 hours, preferably after 6 hours to 24 hours from the administration of the nanoparticles as the probe for molecular imaging. If the time is shorter than the above range, a clear distinction between a lesion site and other sites tends to be difficult. On the other hand, if the time is longer than the above range, the probe for molecular imaging tends to be excreted from a lesion site.

Due to the specific accumulation property as described above, the molecular imaging means makes it possible to, when a vascular lesion site is treated with local therapy after acquisition of image data, more accurately determine the range of the vascular lesion site that should be treated with local therapy and its outer edge. On the other hand, when image data is acquired after local therapy and before internal radiation therapy, the accumulating property of the nanoparticle as an internal radiation therapeutic agent can be predicted in advance, and therefore guidelines for dose and timing of the administration of the internal radiation therapeutic agent can be obtained.

[2-2. Means for positioning needle to be punctured at vascular lesion site based on image data]

The puncture control means, that is, the means for positioning a needle, which should be punctured into a vascular lesion site, at the vascular lesion site based on image data may be a means commonly used in percutaneous local therapy.

Percutaneous local therapy can necrotize at least part, preferably most, of a vascular lesion site that should be treated, and the necrotic portion is inflamed due to a therapeutic effect. That is, percutaneous local therapy is performed to further induce neo vessels caused by inflammation in a vascular lesion site where many neo vessels are present. On the other hand, the nanoparticle as an internal radiation therapeutic agent in the present invention has the effect of specifically accumulating in an angiogenesis site. Therefore, previous percutaneous local therapy performed on a vascular lesion site makes it possible not only to reduce the size of the vascular lesion site but also to further enhance the accumulation property of the nanoparticles, which will be administered later as an internal radiation therapeutic agent, in the vascular lesion site as compared to the case where percutaneous local therapy is not performed. This makes it possible to reduce the radiation value of the internal radiation therapeutic agent to be administered.

Examples of the percutaneous local therapy used in the present invention include percutaneous ethanol injection therapy (PEIT), cryotherapy, radiofrequency ablation (RFA), microwave coagulation therapy (MCT) and the like.

Therefore, the needle to be punctured into a vascular lesion site may be hollow or solid, and may be selected from the group consisting of an injection needle to supply ethanol (which is used for PEIT), an injection needle to supply gas (which is used for cryotherapy), a radiofrequency electrode needle (which is used for RFA), and a microwave electrode needle (which is used for MCT).

It is to be noted that in use of the therapeutic system according to the present invention, PEIT is preferably used in the case of treating a small animal, and RFA is preferably used in the case of treating a large animal.

The means for positioning a needle at a lesion site may include, in addition to the above-described needle, an image-acquiring device for monitoring the movement state of the needle during percutaneous local therapy. The image-acquiring device may be a device which can acquire the image of the needle and tissue into which the needle is punctured per predetermined unit of time. Specifically, the above-described means for acquiring image data showing the position of a vascular lesion site, preferably an ultrasonic diagnostic imaging system or an MRI system may be used.

The image for monitoring the movement state of the needle makes it possible to confirm the puncture position,

direction and the like of the needle. This also makes it possible to, for example, confirm the presence or absence of the positional displacement of the needle tip or a site that needs to avoid puncture.

The means for positioning a needle at a lesion site may further include a puncture control device for controlling the movement of the needle. The puncture control device is used to control the movement of the needle so that the needle can reliably reach a vascular lesion site. For example, the puncture control device may be one that can appropriately correct the direction of the needle tip inserted by an operator when the needle tip travels to a direction different from the direction of a desired vascular lesion site.

Examples of the control of movement of the needle include the determination of the course of the needle (i.e., the determination of which direction the needle is moved) and the determination of the travel of the needle (i.e., the determination of how much the needle is moved). These determinations can be made by, for example, deriving force information suitable for the movement of the needle from force information acquired by a force sensing means that may be provided to detect an external force exerted on the needle. In this case, visual information acquired by the above-described image-acquiring device may be used in combination with the force information, if necessary. A specific means for making the determinations can be appropriately embodied by those skilled in the art.

More specifically, the above determinations can be made by, for example, previously storing general information about tissue acquired as clinically empirical values (e.g., size, shape, elastic modulus, coefficient of friction, shear modulus, Poisson's ratio of human tissue) in an information storing means; allowing a correcting means to derive force information suitable for the movement of the needle by comparing force information acquired by the above-described force sensing means with the above-described general information; and allowing a drive instructing means to give an instruction to drive the needle (an instruction to travel, an instruction to stop, and an instruction to put a resistance load on travel) to convert the above-described force information to a physical momentum.

After the needle to be punctured is positioned at the vascular lesion site in such a manner as described above, predetermined percutaneous local therapy can be performed on the vascular lesion site using the needle. That is, ethanol can be injected in the case of PEIT, liquefied gas or the like can be ejected in the case of cryotherapy, radiofrequency irradiation can be performed in the case of RFA, and microwave irradiation can be performed in the case of MCT. The amounts of ethanol and gas to be injected and ejected, and the doses of radiofrequency wave and microwave can be appropriately determined by those skilled in the art.

[3. Nanoparticle]

The nanoparticle as an internal radiation therapeutic agent in the present invention is a structure having at least a molecular assembly (lactosome) formed by aggregation or self-assembling orientation and association of an amphiphilic block polymer as a carrier agent, and a β-ray emitting nuclide-labeled substance.

One specific aspect of the nanoparticle in the present invention is a molecular assembly formed of the amphiphilic block polymer and the β-ray emitting nuclide-labeled substance.

The molecular assembly in the present invention is formed as a micelle. The amphiphilic block polymer self-assembles so that its hydrophobic block chain forms a core part. On the other hand, the β-ray emitting nuclide-labeled substance may be located in the hydrophobic core.

[3-1. Amphiphilic block polymer]

An amphiphilic block polymer in the present invention has the following hydrophilic block and hydrophobic block. The amphiphilic block polymer is a fundamental element of the molecular assembly as the carrier agent of the nanoparticle. The amphiphilic block polymer can be used singly or in combination of two or more from the amphiphilic block polymers described below. Hereinbelow, in the present invention, the term "amino acid" is used as a concept including natural amino acids, unnatural amino acids, and derivatives thereof by modification and/or chemical alteration. Further, in the specification, amino acids include α-, β-, and γ-amino acids. Among them, α-amino acids are preferred.

[3-1-1. Hydrophilic block chain]

In the present invention, the specific degree of the physical property "hydrophilicity" of a hydrophilic block chain is not particularly limited, but, at least, the hydrophilic block chain shall be hydrophilic enough to be a region relatively more hydrophilic than a specific hydrophobic block chain that will be described later so that a copolymer composed of the hydrophilic block chain and the hydrophobic block chain can have amphiphilicity as a whole molecule of the copolymer, or so that the amphiphilic block polymer can self-assemble in a solvent to form a self-assembly, preferably a particulate self-assembly.

The hydrophilic block chain is a hydrophilic molecular chain comprising a sarcosine-derived unit as an essential hydrophilic structural unit, and having, for example, 20 or more of the essential hydrophilic structural units. More specifically, the hydrophilic molecular chains include: a hydrophilic polypeptide chain having 20 or more, preferably 30 or more sarcosine units.

Sarcosine is N-methylglycine.

When the hydrophilic block chain has a structural unit other than the sarcosine unit, such a structural unit is not particularly limited and examples thereof include an amino acid unit (including hydrophilic amino acids and other amino acids) other than sarcosine unit, and an alkylene oxide unit. Such an amino acid unit is preferably an $\alpha$-amino acid. Examples of the $\alpha$-amino acid include serine, threonine, lysine, aspartic acid, and glutamic acid. Specific examples of the alkylene oxide unit include an ethylene oxide unit (polyethylene glycol unit), a propylene oxide unit (propylene glycol), and the like. In the alkylene oxide unit, hydrogen may be substituted.

In the hydrophilic block chain, the kind and ratio of the structural unit constituting the hydrophilic block chain are appropriately determined by those skilled in the art so that the block chain can have such hydrophilicity as described above as a whole.

The hydrophilic block chain can be designed so that the upper limit of the number of structural units is, for example, about 500. In the present invention, a hydrophilic block chain whose number of structural units is about 30 to 300, preferably about 50 to 200 may be often synthesized. If the number of structural units exceeds about 500, when a molecular assembly is formed, the resultant molecular assembly tends to be poor in stability. If the number of structural units is less than 30, formation of a molecular assembly tends to be difficult per se.

In the hydrophilic block chain, all the same structural units may be continuous or discontinuous. When the hydrophilic block chain contains another structural unit other than the above-described specific units, the kind and ratio of the another structural unit are appropriately determined by those skilled in the art so that the block chain can have the above-described hydrophilicity as a whole. In this case, molecular design is preferably performed so that basic characteristics that will be described later are not impaired.

Sarcosine (i.e., N-methylglycine) is highly water-soluble, and a sarcosine polymer has an N-substituted amide and therefore can be cis-trans isomerized as compared to a normal amide group, and has high flexibility due to less steric hindrance around the $C^{\alpha}$ carbon atom. The use of such a polypeptide as a structural block chain is very useful in that the block chain can have, as basic characteristics, both high hydrophilicity and high flexibility.

[3-1-2. Hydrophobic block chain]

In the present invention, the specific degree of the physical property "hydrophobicity" of a hydrophobic block chain is not particularly limited, but, at least, the hydrophobic block chain shall be hydrophobic enough to be a region relatively more hydrophobic than the hydrophilic block chain so that a copolymer composed of the hydrophobic block chain and the hydrophilic block chain can have amphiphilicity as a whole molecule of the copolymer, or so that the amphiphilic block polymer can self-assemble in a solvent to form a self-assembly, preferably a particulate self-assembly.

In the present invention, the hydrophobic block chain has, for example, 10 or more lactic acid units (in this specification, such a hydrophobic block chain having a lactic acid unit as a base unit is sometimes simply referred to as a polylactic acid) . Preferably, the hydrophobic block chain has 20 or more lactic acid units. In this hydrophobic block chain, all the lactic acid units may be continuous or discontinuous. In the hydrophobic molecular chain, the kind and ratio of a structural unit other than the lactic acid unit are appropriately determined by those skilled in the art so that the block chain can have the above-described hydrophobicity as a whole.

When the hydrophobic block chain has a structural unit other than the lactic acid unit, the kind and ratio of such a structural unit are not particularly limited as long as the block chain has the above-described hydrophobicity as a whole, but the hydrophobic block chain is preferably molecularly-designed so as to have various characteristics that will be described later.

When the hydrophobic block chain has a structural unit other than the lactic acid unit, such a structural unit can be selected from the group consisting of hydroxylic acids other than lactic acid and amino acids (including hydrophobic amino acids and other amino acids) . Examples of the hydroxylic acids include, but are not particularly limited to, glycolic acid, hydroxyisobutyric acid and the like. Many of the hydrophobic amino acids have an aliphatic side chain, an aromatic side chain, or the like. Examples of natural amino acids include glycine, alanine, valine, leucine, isoleucine, proline, methionine, tyrosine, tryptophan and the like. Examples of unnatural amino acids include, but are not particularly limited to, amino acid derivatives such as glutamic acid methyl ester, glutamic acid benzyl ester, aspartic acid methyl ester, aspartic acid ethyl ester, and aspartic acid benzyl ester.

The upper limit of the number of structural units of the hydrophobic block chain is not particularly limited, but is about 100. In the present invention, a hydrophobic block chain whose number of structural units is about 10 to 80, preferably about 20 to 50 may be often synthesized. If the number of structural units exceeds about 100, when a molecular assembly is formed, the formed molecular assembly tends to lack stability. On the other hand, if the number of structural units is less than 10, formation of a molecular assembly tends to be difficult per se.

Polylactic acid has excellent biocompatibility and stability. Therefore, a molecular assembly obtained from the

amphiphilic material containing polylactic acid as a constituent block is very useful from the viewpoint of applicability to a living body, especially a human body.

Further, polylactic acid is rapidly metabolized due to its excellent biodegradability, and is therefore less likely to accumulate in tissue other than vascular lesion site in a living body. Therefore, a molecular assembly obtained from the amphiphilic material containing polylactic acid as a constituent block is very useful from the viewpoint of specific accumulation in vascular lesion site.

Further, polylactic acid is excellent in solubility in low-boiling point solvents. This makes it possible to avoid the use of a hazardous high-boiling point solvent when a molecular assembly is produced from the amphiphilic material containing polylactic acid as a constituent block. Therefore, such a molecular assembly is very useful from the viewpoint of safety for a living body.

Further, adjustment of the chain length of polylactic acid is preferred in that the adjustment contributes, as one factor, to the control of the shape and size of a molecular assembly produced from the amphiphilic material containing polylactic acid as a constituent block. Therefore, the use of such a constituent block is very useful in that a shape of the resulting molecular assembly can give an excellent versatility.

Also when the hydrophobic block chain has a structural unit other than the lactic acid unit, the hydrophobic block chain is preferably molecularly-designed so as to have these various excellent characteristics.

From the viewpoint of optical purity, the hydrophobic block chain may include the following variations.

For example, the lactic acid units constituting the hydrophobic block chain may include only L-lactic acid units, or may include only D-lactic acid units, or may include both L-lactic acid units and D-lactic acid units. The hydrophobic block chain may be used singly or in combination of two or more of them selected from the above examples.

In a case where the lactic acid units include both L-lactic acid units and D-lactic acid units, the order of polymerization of L-lactic acid units and D-lactic acid units is not particularly limited. For example, L-lactic acid units and D-lactic acid units may be polymerized so that one or two L-lactic acid units and one or two D-lactic acid units are alternately arranged, or may be randomly polymerized, or may be block-polymerized.

Therefore, in a case where the lactic acid units include both L-lactic acid units and D-lactic acid units, the amount of each of the lactic acid units is not particularly limited. That is, the amount of L-lactic acid units contained in the hydrophobic block chain and the amount of D-lactic acid units contained in the hydrophobic block chain may be different from each other, or may be the same, and in this case the 10 or more lactic acid units may be a racemate having an optical purity of 0% as a whole.

[3-2. β-ray emitting nuclide-labeled substance]

The β-ray emitting nuclide-labeled substance may be selected from the group consisting of an iodine 131-labeled substance, an yttrium 90-labeled substance, and a lutetium 177-labeled substance.

The β-ray emitting nuclide-labeled substance may be an element encapsulated in the carrier agent or an element constituting part of the carrier agent.

When the β-ray emitting nuclide-labeled substance is an element encapsulated in the carrier agent, the β-ray emitting nuclide-labeled substance is specifically selected from one in which a β-ray emitting nuclide-containing group is bonded to a polymer (β-ray emitting nuclide-labeled polymer), and one in which a β-ray emitting nuclide-containing group is bonded to a hydrophobic compound (β-ray emitting nuclide-labeled compound).

When the β-ray emitting nuclide-labeled substance is an element constituting part of the carrier agent, the β-ray emitting nuclide-labeled substance may specifically be one in which a β-ray emitting nuclide-containing group is bonded to the above-described amphiphilic block polymer. The binding site is not particularly limited, but may preferably be the hydrophilic block chain-side terminal.

The β-ray emitting nuclide in the β-ray emitting nuclide-containing group is a β-ray source for internal radiation therapy of the nanoparticle according to the present invention. The β-ray emitting nuclide has the biological effect of destroying cells or tissue.

When the β-ray emitting nuclide-labeled substance is an element encapsulated in the carrier agent, the β-ray emitting nuclide-containing group is not particularly limited and may be a group chemically or biochemically acceptable in terms of molecular design so that the β-ray emitting nuclide-labeled substance has, as a whole, hydrophobicity that meets the above-described definition of hydrophobicity. When the β-ray emitting nuclide-labeled substance constitutes part of the carrier agent, the β-ray emitting nuclide-containing group is not particularly limited and may be a group chemically or biochemically acceptable in terms of molecular design so that self-assembly of the amphiphilic block polymer is not inhibited. Therefore, the specific structure of the β-ray emitting nuclide-containing group is appropriately determined by those skilled in the art.

The β-ray emitting nuclide-labeled polymer is preferably a β-ray emitting nuclide-labeled polylactic acid.

The polylactic acid group in the β-ray emitting nuclide-labeled polylactic acid is a group whose main structural component is a lactic acid unit. All the lactic acid units may be either continuous or discontinuous. Basically, the structure

or chain length of the polylactic acid group can be determined based on the same viewpoint as in the molecular design of the hydrophobic block chain constituting the amphiphilic block polymer as described above. This makes it possible to obtain the effect that affinity between the β-ray emitting nuclide-labeled polylactic acid and the hydrophobic block chain of the amphiphilic block polymer in the nano-particle is excellent.

The number of lactic acid units of the polylactic acid group is 5 to 50, preferably 15 to 35. The polylactic acid-bound compound is molecularly designed within the above range so that the entire length of the polylactic acid-bound compound does not exceed the length of the above-described amphiphilic block polymer. Preferably, the polylactic acid-bound compound is molecularly designed so that its entire length does not exceed a length of twice the length of the hydrophobic block in the amphiphilic block polymer. If the number of structural units exceeds the above range, when a molecular assembly is formed, the resulting molecular assembly
tends to be poor in stability. If the number of structural units is less than the above range, it tends to be difficult to control the particle size.

From the viewpoint of optical purity, the polylactic acid group may include the following variations.

For example, the lactic acid units constituting the polylactic acid group may include only L-lactic acid units, or may include only D-lactic acid units, or may include both L-lactic acid units and D-lactic acid units. The polylactic acid group may be used singly or in combination of two or more of them selected from the above examples.

In a case where the lactic acid units include both L-lactic acid units and D-lactic acid units, the order of polymerization of L-lactic acid units and D-lactic acid units is not particularly limited. For example, L-lactic acid units and D-lactic acid units may be polymerized so that one or two L-lactic acid units and one or two D-lactic acid units are alternately arranged, or may be randomly polymerized, or may be block-polymerized.

Therefore, in a case where the lactic acid units include both L-lactic acid units and D-lactic acid units, the amount of each of the lactic acid units is not particularly limited.
That is, the amount of L-lactic acid units contained in the hydrophobic block chain and the amount of D-lactic acid units contained in the hydrophobic block chain may be different from each other, or may be the same, and in the latter case the 10 or more lactic acid units may be a racemate having an optical purity of 0% as a whole.

As an example of the β-ray emitting nuclide-labeled polylactic acid, one example of the structure of an iodine 131-labeled polylactic acid is represented by the following formula. The β-ray emitting nuclide can be changed from iodine 131 to another β-ray emitting nuclide by those skilled in the art. In the iodine 131-labeled polylactic acid represented by the following formula, an iodine 131-containing group is introduced into polylactic acid via an amide bond. In the following formula, $R_1$ represents a bivalent organic group. The bivalent organic group $R_1$ can be selected from the group consisting of a bivalent hydrocarbon group and an amide group. The bivalent hydrocarbon group can be selected from the group consisting of an alkylene group that has 3 to 20 carbon atoms and may be substituted and an arylene group that may be substituted. For example, the bivalent organic group $R_1$ may be a group in which an alkylene group and an arylene group are linked by an amide group. The arylene group is preferably a phenylene group. n is an integer of 5 to 50, preferably 15 to
35.

[Chemical formula 1]

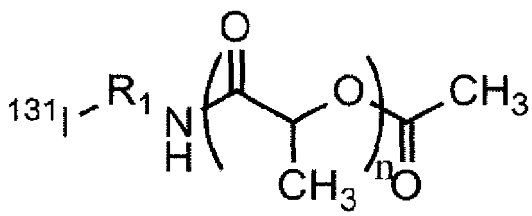

The above-described iodine 131-labeled polylactic acid can be synthesized by reacting an active ester having an iodine 131-containing group with polylactic acid whose one end is aminated. It is to be noted that the active ester having an iodine 131-contaiing group can be obtained by subjecting a tin-containing carboxylic acid to a tin-iodine exchange reaction using an iodinating agent such as $Na^{131}I$ to perform labeling with iodine-131 and then performing active esterification using an active esterifying agent.

A more specific example of the structure of the iodine 131-labled substance that can be synthesized by the above-described method is represented by the following formula. In the following formula, the above bivalent organic group $R_1$ is represented by $-R_1'CONH-C_2H_4-$, and $R_1'$ represents an organic group. The bivalent organic group $R_1'$ may be a bivalent hydrocarbon group. The bivalent hydrocarbon group can be selected from the group consisting of an alkylene group that has 3 to
20 carbon atoms and may be substituted and an arylene group that may be substituted. The arylene group is preferably

a phenylene group. n is an integer of 5 to 50, preferably 15 to 35.

[Chemical formula 2]

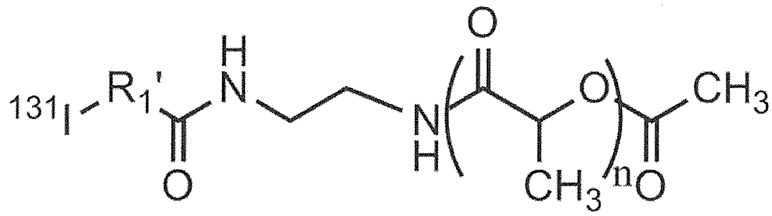

Further, another example of the structure of the iodine 131-labeled polylactic acid is represented by the following formula. In the following formula, $R_2$ represents an organic group. The organic group $R_2$ may be a hydrocarbon having 1 to 18 carbon atoms. Preferably, the organic group $R_2$ may be selected from the group consisting of an alkyl group that has 3 to 6 carbon atoms and may be substituted and an aryl group that may be substituted. The aryl group is preferably a phenyl group. n is an integer of 5 to 50, preferably 15 to 35.

[Chemical formula 3]

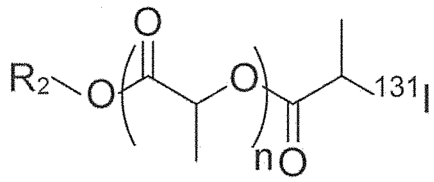

The above iodine 131-labeled polylactic acid can be synthesized by converting another end of polylactic acid whose one end is protected with $R_2$ to a sulfonic acid ester (e.g., a trifluoromethanesulfonic acid ester, a p-toluenesulfonic acid ester or the like) and then performing a 131-iodination reaction using an iodinating agent such as $Na^{131}I$.

[3-3. Content of β-ray emitting nuclide-labeled substance]

The nanoparticles (which have the β-ray emitting nuclide-labeled substance) in the system according to the present invention are prepared in the form of a mixture with nanoparticles having no β-ray emitting nuclide-labeled substance. The amount of the nanoparticles (which have the β-ray emitting nuclide-labeled substance) in the present invention is much smaller than that of nanoparticles having no β-ray emitting nuclide-labeled substance.

For example, in a case where the iodine 131-labeled polylactic acid and the amphiphilic block polymer are used to prepare a nanoparticle mixture, the molar ratio between the iodine 131-labeld polylactic acid and the amphiphilic block polymer is about 1 : 10,000. One nanoparticle is usually composed of about 200 molecules of the amphiphilic block polymer, and 1 molecule of the β-ray emitting nuclide-labeled substance is encapsulated in one nanoparticle. Therefore, the ratio of the number of the nanoparticles (which have the β-ray emitting nuclide-labeled substance) in the present invention to the number of nanoparticles having no β-ray emitting nuclide-labeled substance may be 1 : 50.

[3-4. Size of nanoparticle]

The size of the nanoparticle in the present invention is, for example, 10 to 500 nm, preferably 20 to 200 nm. Here, the term "particle size" refers to a particle diameter that appears at the highest frequency in a particle size distribution, that is, a median particle diameter. A nanoparticle having a particle size smaller than 10 nm is difficult to be produced, or said nanoparticle tends to be less likely to accumulate in a vascular lesion site. A nanoparticle having a particle size larger than 500 nmmay not be suitable as an injection product when administered to a living body by injection.

A method for measuring the size of the nanoparticle in the present invention is not particularly limited, and is appropriately selected by those skilled in the art. Examples of such a method include an observation method using a transmission electron microscope (TEM) or an atomic force microscope (AFM), and a dynamic light scattering (DLS) method. In the DLS method, the translational diffusion coefficient of a particle undergoing Brownian movement in a solution is measured.

An example of a method for controlling the size of the nanoparticle is a method in which the length of the

amphiphilic block polymer is adjusted. Another example of the method is a method in which when the β-ray emitting nuclide-labeled substance is a β-ray emitting nuclide-labeled polymer, the length of the polymer group is adjusted.

[3-5. Formation of nanoparticle]

A method for forming the nanoparticle is not particularly limited, and can be appropriately selected by those skilled in the art depending on the desired size and characteristics of the nanoparticle; the kind, properties and content of the β-ray emitting nuclide-labeled substance to be encapsulated; or the like. If necessary, after nanoparticles are formed in the following manner, the obtained nanoparticles may be subjected to surface modification by a known method.

It is to be noted that the confirmation of formation of particles may be performed by electron microscope observation.

[3-5-1. Film method]

A film method is a method that has been used for liposome preparation. The amphiphilic block polymer in the present invention has solubility in a low boiling point solvent, and therefore the nanoparticle can be prepared by this method.

The film method comprises the following steps of: preparing a solution, in a container (e.g., a glass container), containing the amphiphilic block polymer and the β-ray emitting nuclide-labeled substance in an organic solvent; removing the organic solvent from the solution to obtain, on an inner wall of the container, a film containing the amphiphilic block polymer and the β-ray emitting nuclide-labeled substance; and adding water or an aqueous solution to the container, and performing ultrasonic treatment, warming treatment, or both of the treatments to convert the film-shaped substance into molecular assemblies including the β-ray emitting nuclide-labeled substance to obtain a dispersion liquid of nanoparticles. Further, this film method may comprise the step of subjecting the dispersion liquid of nanoparticles to freeze-drying treatment.

The solution containing the amphiphilic block polymer and the β-ray emitting nuclide-labeled substance in an organic solvent may be prepared by previously preparing a film comprising the amphiphilic block polymer, and then adding a solution containing the β-ray emitting nuclide-labeled substance at the time of nanoparticle preparation to the film for dissolution.

The organic solvent to be used in the film method is preferably a low boiling point solvent. In the present invention, the low boiling point solvent refers to a solvent whose boiling point at 1 atmospheric pressure is 100°C or lower, preferably 90°C or lower. Specific examples of the low boiling point solvent include chloroform, diethyl ether, acetonitrile, methanol, ethanol, acetone, dichloromethane, tetrahydrofuran, hexane, and the like.

The use of such a low boiling point solvent to dissolve the amphiphilic block polymer and the β-ray emitting nuclide-labeled substance makes it very easy to perform solvent removal. A method for solvent removal is not particularly limited, and may be appropriately determined by those skilled in the art depending on the boiling point of an organic solvent to be used, or the like. For example, solvent removal may be performed under reduced pressure, or by natural drying.

After the organic solvent is removed, a film containing the amphiphilic block polymer and the β-ray emitting nuclide-labeled substance is formed on the inner wall of the container. Water or an aqueous solution is added to the container to which the film is attached. The water or aqueous solution is not particularly limited, and biochemically or pharmaceutically acceptable ones may be appropriately selected by those skilled in the art. Examples thereof include distilled water for injection, normal saline, and a buffer solution.

After water or an aqueous solution is added, warming treatment is performed. The film is peeled off from the inner wall of the container by warming, and in this process, molecular assemblies are formed. The warming treatment can be performed under the conditions of, for example, 70 to 100°C and 2 to 60 minutes. After the completion of the warming treatment, a dispersion liquid in which molecular assemblies (nanoparticles) encapsulating the β-ray emitting nuclide-labeled substance are dispersed in the water or aqueous solution is prepared in the container.

The obtained dispersion liquid can be directly administered to a living body. That is, the nanoparticles do not need to be stored by themselves under solvent-free conditions.

On the other hand, the obtained dispersion liquid may be subjected to freeze-drying treatment. A method for freeze-drying treatment is not particularly limited, and any known method can be used. For example, the dispersion liquid of nanoparticles obtained in such a manner as described above may be frozen by liquid nitrogen, or the like, and sublimated under reduced pressure. In this way, a freeze-dried product of the nanoparticles is obtained. That is, the nanoparticles can be stored as a freeze-dried product. If necessary, water or an aqueous solution may be added to the freeze-dried product to obtain a dispersion liquid of nanoparticles, and the nanoparticles can be used. The water or aqueous solution is not particularly limited, and biochemically or pharmaceutically acceptable ones may be appropriately selected by those skilled in the art. Examples thereof include distilled water for injection, normal saline, and a buffer

solution.

Here, the dispersion liquid before freeze-drying treatment may contain, in addition to the nanoparticles according to the present invention formed from the amphiphilic block polymer and the β-ray emitting nuclide-labeled substance, the amphiphilic block polymer and/or the β-ray emitting nuclide-labeled substance remaining per se without contributing to the formation of such nanoparticles. By subjecting such a dispersion liquid to freeze-drying treatment, in the process of concentration of a solvent, it is possible to further form nanoparticles from the amphiphilic block polymer and the β-ray emitting nuclide-labeled substance remaining without forming the nanoparticles according to the present invention. Therefore, preparation of the nanoparticles according to the present invention can be efficiently performed.

[3-5-2. Injection method]

An injection method is a method used for preparation of not only the nanoparticle according to the present invention but also many other nanoparticles. In this method, the amphiphilic block polymer and the β-ray emitting nuclide-labeled substance are dissolved in an organic solvent such as trifluoroethanol, methanol, ethanol, hexafluoroisopropanol, dimethylsulfoxide, dimethylformamide, or the like to obtain a solution; and the solution is dispersed in a water-based solvent such as distilled water for injection, normal saline, or a buffer solution and subjected to purification treatment such as gel filtration chromatography, filtering, or ultracentrifugation; and then the organic solvent is removed to prepare nanoparticles. When nanoparticles obtained in this way using an organic solvent hazardous to a living body are administered to a living body, the organic solvent needs to be strictly removed.

[3-6. Administration of nanoparticle]

When the therapeutic system according to the present invention is used, a method for administering the nanoparticles into a living body is not particularly limited and can be appropriately determined by those skilled in the art. Therefore, the administration method may be either systemic administration or local administration. That is, the administration can be also performed by any one of injection (needle injection or needleless injection), infusion, oral administration, or external application.

The nanoparticles as an internal radiation therapeutic agent are preferably administered after neo vessels are adequately induced by percutaneous local therapy. For example, the nanoparticles can be administered after 1 hour to 168 hours, or 12 hours to 72 hours, e.g., 24 hours from the completion of percutaneous local therapy.

The amount of the nanoparticles administered as an internal radiation therapeutic agent can be appropriately determined by those skilled in the art after the confirmation of accumulating property of the nanoparticle in a lesion, which should be treated with internal radiation therapy, with the use of a probe for molecular imaging. As the probe for molecular imaging preferably used for the confirmation of the accumulating property of the nanoparticle, as described in item 2-1, a nanoparticle is used which contains, as a carrier agent, the same carrier agent as the nanoparticle as an internal radiation therapy and contains, as a substance encapsulated in the carrier agent, a substance labeled with a radioisotope for molecular imaging. In this case, the nanoparticle as a probe for molecular imaging and the nanoparticle as an internal radiation therapeutic agent use the same carrier agent, and therefore their accumulating property can be considered the same.

The nanoparticles as an internal radiation therapeutic agent in the system according to the present invention are prepared depending on the species of an individual as an object to which the nanoparticles are administered so that their radiodensity is sufficient to destroy cells or tissue in a lesion and is acceptable for the species of the individual.

The nanoparticles in the present invention can be administered in a dose equivalent to that of, for example, a radioactive iodine 131-containing therapeutic agent for use in conventional therapy of thyroid cancer or Graves' disease or an yttrium 90-labeled anti-CD20 antibody for use in conventional therapy of malignant lymphoma.

It is considered that a tumor growth-suppressing effect can be obtained by the accumulation of about 0.25 MBq of the nanoparticles in the present invention in a lesion site. On the other hand, when the nanoparticles are administered at more than 15 MBq per mouse (25 g), there may be a case where the mouse dies from significant side effects of radiation. In consideration of the above descriptions, when the system according to the present invention is applied to a mouse, the nanoparticles can be prepared so as to have a radiation value of 10 MBq/kg to 600 MBq/kg for one-time use.

When administered, the nanoparticles in the present invention may be prepared as an injection solution in which the nanoparticles are dissolved or dispersed in a pharmaceutically-acceptable buffer solution, e.g., sterile water for injection (BWFI), phosphate buffer saline, Ringer's liquid, dextrose solution or the like. The injection solution may contain the nanoparticles (which have the β-ray emitting nuclide-labeled substance) in the present invention and nanoparticles having no β-ray emitting nuclide-labeled substance in a number ratio of about 1 : 50.

EXAMPLES

Hereinbelow, the present invention will be described in more detail with reference to examples, but is not limited thereto.

[Experimental Example 1: Synthesis of [$^{131}$I]-SIB]

In this experimental example, $^{131}$I-SIB (N-succinimidyl 4-[$^{131}$I]iodobenzoate) was synthesized from a tin precursor.

[Chemical formula 4]

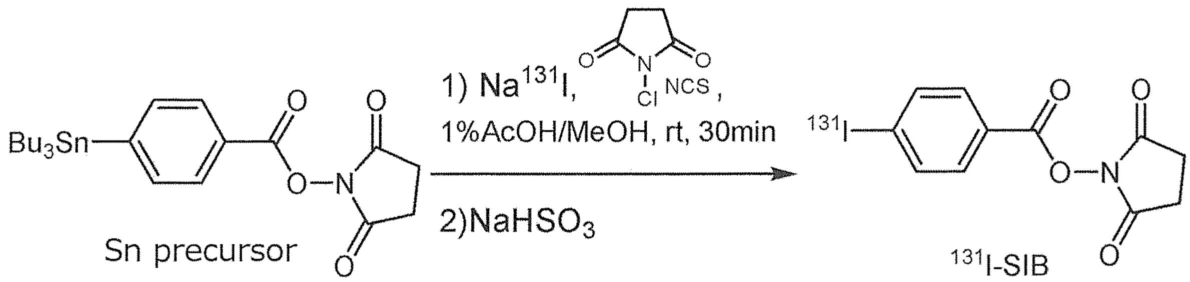


A solution was prepared in which 4-tributyltin benzoate succinimidyl ester (tin precursor) had been previously dissolved in a methanol solution containing 1 (v/v) % of acetic acid, and was mixed with an aqueous Na$^{131}$I solution (354. 1 MBq) . The solution was slightly in a clouded state immediately after mixing, but was immediately returned to a colorless and transparent state by stirring. A methanol solution of N-chlorosuccinimide (NCS) was added thereto to perform a reaction at room temperature. After 30 minutes from the start of the reaction, sodium hydrogen sulfite was added to the reaction solution to quench the reaction, and then the total amount of the obtained reaction mixture solution was injected into a reversed-phase HPLC system to purify and collect a target substance. HPLC charts obtained at this time are shown in Fig. 1. Fig. 1(a) shows the elution chart of a radioisotope (i.e., $^{131}$I) (horizontal axis: elution time (min), vertical axis: detected intensity (mV)), and Fig. 1(b) shows the elution chart of a substance (i.e., SIB) at a wavelength of 254 nm (horizontal axis: elution time (min), vertical axis: detected intensity (mV)). A substance eluted at 10.0 minutes to 13.5 minutes (shown as shaded area) in Fig. 1(a) and at 9.5 minutes to 12.5 minutes in Fig. 1(b) was $^{131}$I-SIB, and an eluate was collected during this period of time. The collected solution was diluted 10-fold or more with water, passed through Sep-Pak C18, and eluted into about 300 µL of an acetonitrile solution for solid phase extraction. The radiation dose of the collected $^{131}$I-SIB was 163.5 MBq, that is, a yield of 46.2% was achieved.

[Experimental Example 2: Synthesis of Aminated Poly-L-Lactic Acid]

In this experimental example, aminated poly-L-lactic acid (a-PLLA) was synthesized using L-lactide (compound 1) and N-carbobenzoxy-1,2-diaminoethane hydrochloride (compound 2) .

[Chemical Formula 5]

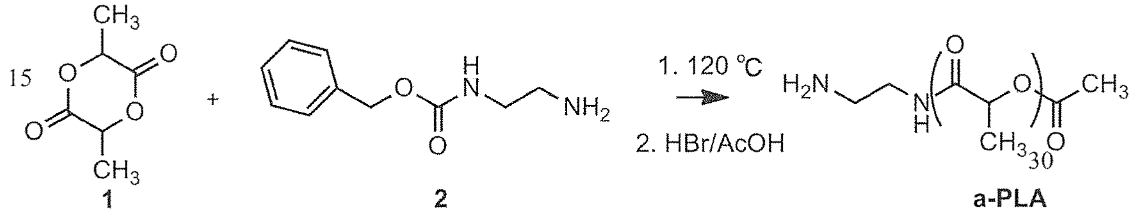


1 2 a-PLA

To N-carbobenzoxy-1,2-diaminoethane hydrochloride (compound 2) (310 mg, 1.60 mmol) served as a polymerization initiator, a dispersion liquid obtained by dispersing tin octanoate (6.91 mg) in toluene (1.0 mL) was added. The toluene was distilled away under reduced pressure, and then L-lactide (compound 1) (3.45 g, 24 mmol) was added to perform polymerization reaction at 120°C under an Ar atmosphere. After 12 hours, the reaction container was air-cooled to room temperature to obtain a yellowish-white solid. The obtained yellowish-white solid was dissolved in a small amount of chloroform (about 10 mL). The resulting chloroform was dropped into cold methanol (100 mL) to obtain a white precipitate. The obtained white precipitate was collected by centrifugation and dried under reduced pressure.

To a dichloromethane (1 mL) solution of the obtained white precipitate (500 mg), 25 v/v% hydrogen bromide/acetic acid (2.0 mL) was added, and the mixture was stirred for 2 hours under dry air atmosphere in a shading environment. After the completion of reaction, the resultant reaction solution was dropped into cold methanol (100 mL) so that a precipitate was deposited. The precipitate was collected by centrifugation. The obtained white precipitate was dissolved in chloroform, washed with a saturated aqueous $NaHCO_3$ solution, and then dehydrated with anhydrous $MgSO_4$. Then, the $MgSO_4$ was removed by Celite® filtration, and the white precipitate was vacuum-dried to obtain white amorphous powder of a-PLLA (440 mg) .

[Experimental Example 3: Synthesis of polysarcosine-polylactic acid amphiphilic block polymer ($PSar_{70}$-$PLLA_{30}$)]

In this experimental example, a polysarcosine-polylactic acid amphiphilic block polymer ($PSar_{70}$-$PLLA_{30}$) was synthesized from sarcosine-NCA (Sar-NCA) and aminated poly-L-lactic acid (a-PLLA).

[Chemical formula 6]

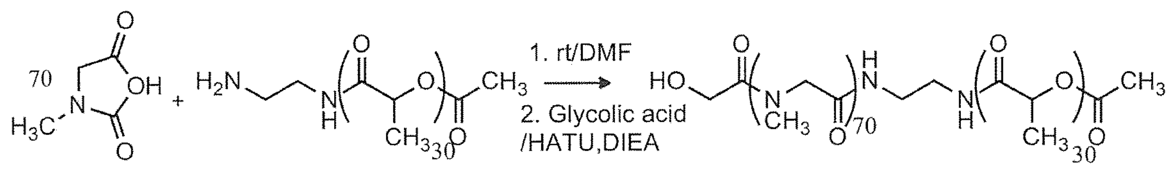


**Sar-NCA** **a-PLA** **$PSar_{70}$-$PLA_{30}$**

Dimethylformamide (DMF) (140 mL) was added to a-PLLA (383 mg, 0.17 mmol) and sarcosine-NCA (Sar-NCA) (3.21 g, 27.9 mmol) under an Ar atmosphere, and the mixture was stirred at room temperature for 12 hours. After the reaction solution was cooled to 0°C, glycolic acid (72 mg, 0.95 mmol), O-(benzotriazol-1-yl)-N,N,N' ,N'-tetramethyluronium hexafluorophosphate (HATU) (357 mg, 0.94 mmol), and N,N-diisopropylethylamine (DIEA) (245 μL, 1.4 mmol) were added to the reaction solution, and reaction was performed at room temperature for 18 hours.

After DMF was distilled away under reduced pressure by a rotary evaporator, purification was performed using an LH20 column. Fractions showing a peak detected at UV 270 nm were collected and concentrated. The thus obtained concentrated solution was dropped into diethyl ether at 0°C for reprecipitation to obtain $PSar_{70}$-$PLLA_{30}$ (1.7 g) as a target substance.

[Experimental Example 4: Synthesis of [$^{131}$I]-$PLLA_{30}$]

In this experimental example, a condensation reaction between $^{131}$I-SIB and aminated poly-L-lactic acid (a-$PLLA_{30}$) was performed.

[Chemical formula 7]

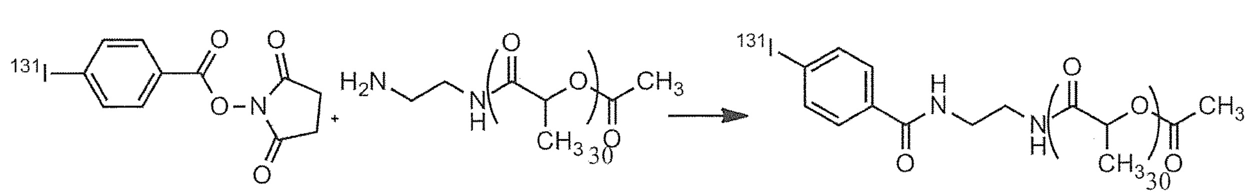

**$^{131}$I-SIB** **a-PLA** **$^{131}$I-BzPLA**

To 300 μL of an acetonitrile solution of $^{131}$I-SIB (163.5 MBq), 100 to 150 μL of a DMSO solution containing 1.5 mg of a-$PLLA_{30}$ was added, and the mixture was heated at 100°C for 20 minutes.

After the completion of the condensation reaction, the reaction mixture solution was subjected to gel permeation chromatography by HPLC to separate and purify a target substance by HPLC. Fig. 2 shows an RI signal chart (horizontal axis: elution time (min), vertical axis: detected intensity (mV)). A major elution peak attributable to $^{131}$I-BzPLLA$_{30}$ appeared in a range from 7.5 minutes to 11.5 minutes (shown as shaded area), and an eluate in this range was collected. The radiation dose of the obtained $^{131}$I-BzPLLA$_{30}$ was 107.7 MBq, that is, a yield of 30.4% was achieved.

[Experimental Example 5: Formation of Particles of $^{131}$I-Lactosome]

The acetonitrile solution of $^{131}$I-BzPLLA$_{30}$ obtained in Experimental Example 4 was mixed with a polymer film using 9 mg of PSar$_{70}$-PLLA$_{30}$, and the mixture was dried by blowing air while heated to 80°C to prepare a film containing $^{131}$I-BzPLLA$_{30}$ and PSar$_{70}$-PLLA$_{30}$. The obtained film was added with normal saline and subjected to ultrasonic treatment at 85°C for 2 minutes, to thereby obtain a dispersion liquid of $^{131}$I-labeled lactosome ($^{131}$I-lactosome) was obtained.

[Experimental Example 6: Synthesis of ICG-labeled polylactic acid]

The aminated poly-L-lactic acid (a-PLA) was labeled with ICG as a fluorescent dye to obtain ICG-labeled poly-L-lactic acid (ICG-PLLA$_{30}$). Specifically, a DMF solution containing 1 mg (1.3 eq) of an indocyanine green derivative (ICG-sulfo-OSu) dissolved therein was added to a DMF solution containing 1.9 mg (1.0 eq) of a-PLA and stirred at room temperature for about 20 hours. Then, the solvent was distilled away under reduced pressure, and purification was performed using an LH20 column to obtain a compound, ICG-PLLA$_{30}$.

[Chemical formula 8]

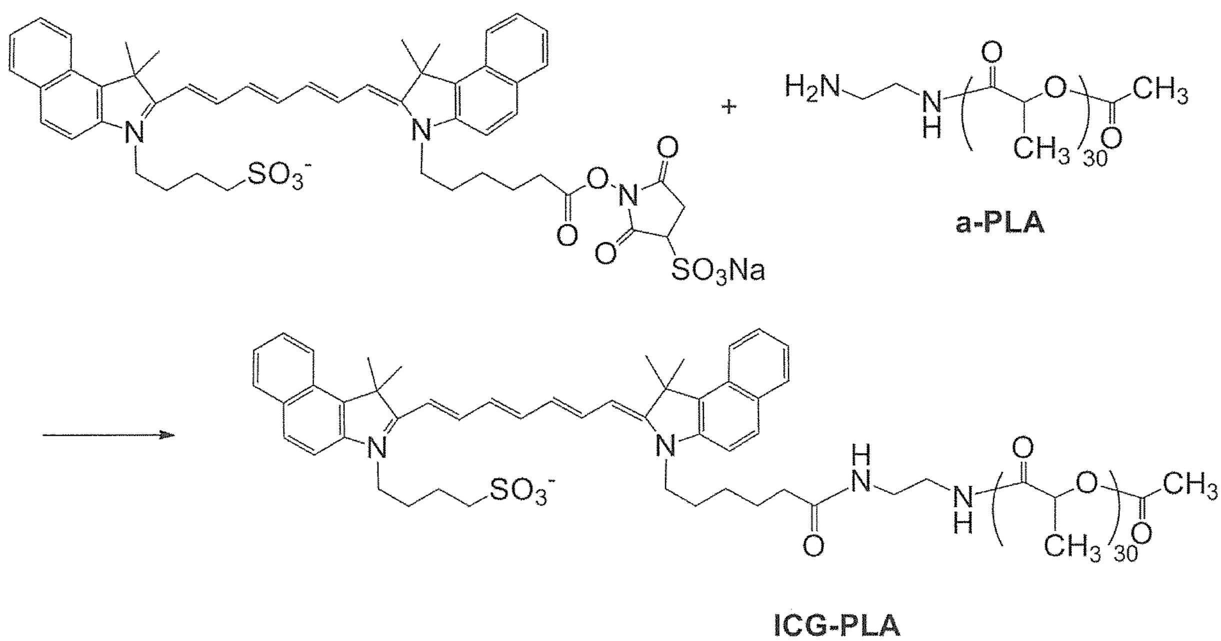


[Experimental Example 7: Formation of Particles of Lactosome Encapsulating ICG-Labeled Polylactic Acid]

A chloroform solution (0.2 mM) of the polylactic acid-polysarcosine amphiphilic block polymer (PSar$_{70}$-PLLA$_{30}$. 26H$_2$O, MW = 7,767) obtained as a carrier agent in Experimental Example 3 was prepared. Further, a chloroform solution (0.2 mM) of the ICG-labeled polylactic acid (PLA-ICG) obtained in Experimental Example 6 was prepared. A mixed solution of both the solutions was prepared in a glass container so that the molarity of a fluorescent dye ICG was 20 mol%. Then, a lactosome was prepared by a film method. It is to be noted that the film method was performed in the following manner. The solvent was distilled away from the mixed solution under reduced pressure to form a film containing the carrier agent and the fluorescent dye on the wall surface of the glass container. Further, water or a buffer solution was added to the glass container having the film formed therein, and the glass container was put in hot water at 82°C for 20 minutes and was then allowed to stand at room temperature for 30 minutes, and the water or buffer solution was filtered with a 0.2 μm filter and freeze-dried.

[Experimental Example 8: Fluorescent Imaging Test of Subcutaneous Cancer Using ICG-Labeled Polylactic Acid-Encapsulating Lactosome]

Cancer-bearing mice were produced by subcutaneous transplantation of mouse cancer cells in the following manner.

As animals, 7-week-old Hairless SCID mice (OrientalBioService, Inc) were used. Each of the mice was anesthetized with Somnopentyl. A mouse breast cancer cell line (4T1) was mixed with a Geltrex matrix gel and subcutaneously transplanted in the left mammary gland of each of the mice at $5 \times 10^5$ cells/0. 02 mL. At the time when the cancer tissue reached a size of 5 mm after growth for 6 days, each of the mice was subjected to inhalation anesthesia, and 0.05 mL of anhydrous ethanol was directly injected into the tumor site. After 7 days from the transplantation, each of the mice was subjected to the following imaging test.

Each of the cancer-bearing mice was anesthetized with isoflurane, and 0.05 mL of a dispersion liquid of a lactosome encapsulating 20 mol% of ICG-$PLLA_{30}$ (0.1 nmol/body) was administered as a molecular probe from its tail vein. After the administration of the lactosome dispersion liquid, fluorescence images of the whole body of each of the mice were taken with time. The fluorescence images of the whole body were taken from five directions, that is, from all the directions of left abdomen, left side of the body, back, right side of the body, and right abdomen of the mouse. The fluorescent dye was excited at 785 nm, and fluorescence at about 845 nm was measured with time.

Fig. 3 shows a comparison between the obtained images and images of a control group (group receiving no PEIT; for comparison). In Fig. 3, the results of measurement performed before the tail-vein administration of the lactosome to each of the mice and after 15 minutes, 1 hour, 3 hours, 6 hours, 9 hours, 24 hours, and 48 hours from the administration to each of the mice are shown in this order from the above. In Fig. 3, high and low in fluorescence intensity are indicated by a difference in color.

Fig. 4 shows the results of changes in fluorescence intensity analyzed from the fluorescence images. Specifically, Fig. 4 shows changes in light intensity in the tumor (tumor), liver (liver), mammary gland opposite to the mammary gland in which the tumor had been transplanted (Background (breast)), and back (Background (back)) of 3 mice of the control group and 4 mice of a PEIT group (horizontal axis: time (time (h)), vertical axis: light intensity (Total Flux).

As shown in Fig. 4, in the case of the control group, the peak of accumulation of the lactosome in the tumor was observed after 9 hours from the administration, and on the other hand, in the case of the PEIT group, the peak of accumulation of the lactosome in the tumor was observed after 48 hours from the administration. It was found that the amount of the lactosome that remained accumulated in the tumor in the PEIT group was about twice that in the control group.

[Experimental Example 9: Results of Measurement of Distribution of $^{131}$I-Lactosome in Body]

Cancer-bearing mice were produced by subcutaneous transplantation of mouse cancer cells in the following manner.

As animals, 7-week-old Hairless SCID mice (OrientalBioService, Inc) were used. Each of the mice was anesthetized with Somnopentyl. A mouse breast cancer cell line (4T1) was mixed with a Geltrex matrix gel and subcutaneously transplanted in the left mammary gland of each of the mice at $5 \times 10^5$ cells/0.02 mL. At the time when the cancer tissue reached a size of 5 mm after growth for 6 days, each of the mice of a PEIT group was anesthetized with Somnopentyl, and 0.05 mL of anhydrous ethanol was directly injected into the tumor site. After 7 days from the transplantation, each of the mice was subjected to autopsy.

To each of the cancer-bearing mice of a control group and a PEIT group, 75 kBq/0.1 mL/body of the $^{131}$I-lacosome was administered from its tail vein. Each group contained 3 mice. After 24 hours, 48 hours, 72 hours, and 168 hours from the administration, autopsy was performed and each of the organs (pancreas, spleen, stomach, small intestine, colon, liver, kidney, lung, heart, muscle, thyroid, tumor, bone, brain, and blood) was collected in a test tube to measure radioactivity derived from 1-131 with a $\gamma$-counter. Fig. 5 shows % ID/g of each of the organs (i.e., % Injected dose/g). However, the values of the stomach and the thyroid are not divided by weight. In the bar graph shown in Fig. 5, bars for each of the organs represent the results of the control group or the PEIT group after 24 hours, 48 hours, 72 hours, and 168 hours from the administration, respectively, from the left side.

It was confirmed from Fig. 5 that there was no change in the amount of 1-131 accumulated in each of the organs, but the amount of I-131 accumulated only in the tumor site after 48 hours and 72 hours from the administration in the PEIT group was confirmed to be about three times that in the control group. Further, after 168 hours from the administration, the % Injected dose/g of the tumor site in the control group was 0.04%, whereas the % Injected dose/g of the tumor site in the PEIT group was 0.98%, that is, the amount of 1-131 accumulated in the tumor site was higher in the PEIT group than in the control group.

[Experimental Example 10: Anticancer Activity Test for $^{131}$I-Lactosome Using Mouse Breast Cancer Cell Line 4T1 Cells]

The anticancer activity of the $^{131}$I-lactosome was tested using mouse breast cancer cell line 4T1 cells in the following manner.

In a 96-well plate, $5 \times 10^2$ 4T1 cells/0.1 mL were cultured at 37°C for 24 hours using 5% FBS-Dulbecco's modified Eagle medium. Then, 10 μL of a lactosome dispersion liquid was added to each of the wells so that the final concentration of the $^{131}$I-lactosome was 15.6 kBq/well to 500 kBq/well, and the 4T1 cells were cultured.

The lactosome dispersion liquid contained the $^{131}$I-lactosome at a predetermined final concentration, and an unlabeled lactosome composed of only $PSar_{70}$-$PLLA_{30}$ and containing no $^{131}$I-$BzPLLA_{30}$, and the total amount of the $^{131}$I-lactosome and the unlabeled lactosome was 0.19 mg/well.

Separately, 90 μL of 5% FBS-Dulbecco's modified Eagle medium and 10 μL of a cell-counting reagent SF (manufactured by NACALAI TESQUE, INC.) were mixed to prepare a mixed liquid.

After lapses of 24 hours, 48 hours, and 72 hours from the start of cultivation, a supernatant was removed from each of the wells, and 0.1 mL of the above mixed liquid was added to each of the wells, and the wells were allowed to stand at 37°C for 2 hours. Then, the absorbance at 450 nm was measured and compared with that of a control containing no reagent. The measurement results are shown in Fig. 6. In Fig. 6, the horizontal axis represents time (H) after addition of the $^{131}$I-lactosome, and the vertical axis represents absorbance (OD at 450 nm) dependent on the number of living cells. It was found that the $^{131}$I-lactosome significantly had a cell growth-suppressing effect at a concentration of 250 kBq/well or higher.

It is to be noted that in Fig. 6, the "Lactosome" means that only an unlabeled lactosome was added, that is, the concentration of the $^{131}$I-lactosome was 0 kBq/well.

[Example 1: Antitumor Test on Mice Using Combination of $^{131}$I-Lactosome Administration and PEIT]

Cancer-bearing mice were produced by subcutaneous transplantation of mouse cancer cells in the following manner.

As animals, 6- to 7-week-old Hairless SCID mice (OrientalBioService, Inc) were used. Each of the mice was anesthetized with Somnopentyl. A mouse breast cancer cell line (4T1) was mixed with a Geltrex matrix gel and subcutaneously transplanted in the left mammary gland of each of the mice at $5 \times 10^5$ cells/0.02 mL. At the time when the cancer tissue reached a size of 5 mm after growth for 6 days, each of the mice of a PEIT group was etherized, and 0.05 mL of anhydrous ethanol was directly injected into the tumor site. After 7 days from the transplantation, each of the mice was subjected to an antitumor test.

The above cancer-bearing mice were divided into a control group (for comparison), a PEIT group (for comparison), a PEIT + lactosome group (for comparison), a PEIT + NaI group (for comparison), and a PEIT + $^{131}$I-lactosome group. Each group contained 5 cancer-bearing mice.

In the control group (for comparison) and the PEIT group (for comparison), 0.1 mL/body of normal saline was administered to each of the cancer-bearing mice from its tail vein; in the PEIT + lactosome group (for comparison), 0.1 mL/body of a lactosome was administered to each of the cancer-bearing mice from its tail vein; in the PEIT + NaI group (for comparison), 5 MBq/0.1 mL/body of NaI was administered to each of the cancer-bearing mice from its tail vein; and in the PEIT + $^{131}$I-lactosome group, 5 MBq/0.1 mL/body of the above-described $^{131}$I-lactosome was administered to each of the cancer-bearing mice from its tail vein. The lactosome administered in the PEIT + lactosome group (for comparison) contained no $^{131}$I-$BzPLLA_{30}$ and was composed of only $PSar_{70}$-$PLLA_{30}$.

After the administration, the tumor volume and the body weight were measured every 2 or 3 days for 16 days. Changes in the tumor volume are shown in Fig. 7, and changes in the body weight are shown in Fig. 8.

It is to be noted that, in Fig. 7, the size of the tumor was measured with a vernier caliper, and the tumor volume was calculated by the equation:

$$\text{Tumor Volume (mm}^3\text{)} = \text{Longer Diameter} \times (\text{Shorter Diameter})^2 / 2 ;$$

and
a relative tumor volume was calculated by the equation:

$$\text{Relative Tumor Volume} = \text{Tumor Volume on Measurement Day} / \text{Tumor Volume on Administration Day};$$

assuming that the tumor volume on the administration day was 1.

It is to be noted that, in Fig. 8, the change in body weight shows an increase or decrease (g) from the administration day, and a body weight gain was calculated by the equation:

Body Weight Gain = Body Weight on Measurement Day - Body Weight on Administration Day.

A statistical significance test was performed using repeated measures (analysis of variance) in JUMP. In the relative tumor volume shown in Fig. 7, a significant antitumor effect was observed in the $^{131}$I-lactosome-administered group as compared to the PEIT group. Further, as a result of repeated measures (analysis of variance) in JUMP, it was confirmed that a significant difference between the PEIT group and the PEIT + $^{131}$I-lactosome group was $p < 0.0001$. Further, as can be seen from the body weight gain shown in Fig. 8, the body weight was not reduced by administration of the $^{131}$I-lactosome.

[Reference Example 1: Antitumor Test on Mice by Administration of $^{131}$I-Lactosome (5 MBq/body) alone / without PEIT]

Cancer-bearing mice were produced by subcutaneous transplantation of mouse cancer cells in the following manner.

As animals, 6- to 7-week-old Hairless SCID mice (OrientalBioService, Inc) were used. Each of the mice was anesthetized with Somnopentyl. A mouse breast cancer cell line (4T1) was mixed with a Geltrex matrix gel and subcutaneously transplanted in the left mammary gland of each of the mice at $5 \times 10^5$ cells/0.02 mL. The cancer tissue reached a size of 5 mm after growth for 6 days, and after 7 days from the transplantation, each of the mice was subjected to an antitumor test.

The above cancer-bearing mice were divided into a control group, a lactosome group, a NaI group, and an $^{131}$I-lactosome group. Each group contained 5 cancer-bearing mice.

In the control group, 0.1 mL/body of normal saline was administered to each of the cancer-bearing mice from its tail vein; in the lactosome group, 0.1 mL/body of a lactosome was administered to each of the cancer-bearing mice from its tail vein; in the NaI group, 5 MBq/0.1 mL/body of NaI was administered to each of the cancer-bearing mice from its tail vein; and in the $^{131}$I-lactosome group, 5 MBq/0.1 mL/body of the above-described $^{131}$I-lactosome was administered to each of the cancer-bearing mice from its tail vein. The lactosome administered in the lactosome group contained no $^{131}$I-BzPLLA$_{30}$ and was composed of only PSar$_{70}$-PLLA$_{30}$.

After the administration, the tumor volume and the body weight were measured every 2 or 3 days for 16 days in the same manner as in Example 1. Changes in the tumor volume are shown in Fig. 9, and changes in the body weight are shown in Fig. 10. A relative tumor volume shown in Fig. 9 and a body weight gain shown in Fig. 10 were also determined in the same manner as in Example 1.

In the relative tumor volume shown in Fig. 9, an antitumor effect was slightly observed in the $^{131}$I-lactosome-administered group as compared to the control group, but was lower than that observed in the PEIT + $^{131}$I-lactosome group in Example 1. As can be seen from the body weight gain shown in Fig. 10, the body weight was not reduced by administration of the $^{131}$I-lactosome.

[Reference Example 2: Antitumor Test on Mice by Administration of $^{131}$I-Lactosome (5 MBq/body) alone / without PEIT]

Cancer-bearing mice were produced by subcutaneous transplantation of human cancer cells in the following manner.

As animals, 7-week-old BALB/c nu/nu mice (SLC) were used. Each of the mice was anesthetized with Somnopentyl. Human pancreas cancer cells (Suit2) were mixed with a Geltrex matrix gel and subcutaneously transplanted in the right arm of each of the mice at $1 \times 10^6$ cells/0.04 mL. At the time when the cancer tissue reached a size of 5 mm after growth for 14 days, each of the mice was subjected to an antitumor test.

The above cancer-bearing mice were divided into a control group, a lactosome group, a NaI group, and an $^{131}$I-lactosome group. Each group contained 8 cancer-bearing mice.

In the control group, 0.1 mL/body of normal saline was administered to each of the cancer-bearing mice from its tail vein; in the lactosome group, 0.1 mL/body of a lactosome dispersion liquid was administered to each of the cancer-bearing mice from its tail vein; in the NaI group, 5 MBq/0.1 mL/body of an aqueous Na$^{131}$I solution was administered to each of the cancer-bearing mice from its tail vein; and in the $^{131}$I-lactosome group, 5 MBq/0.1 mL/body of an $^{131}$I-lactosome dispersion liquid was administered to each of the cancer-bearing mice from its tail vein. The lactosome administered in the lactosome group contained no $^{131}$I-BzPLLA$_{30}$ and was composed of only PSar$_{70}$-PLLA$_{30}$.

After the administration, the tumor volume was measured every 2 or 3 days for 13 days. Changes in the tumor volume are shown in Fig. 11. It is to be noted that, in Fig. 11, the size of the tumor was measured with a vernier caliper, the tumor volume was calculated by the equation:

$$\text{Tumor Volume (mm}^3\text{)} = \text{Longer Diameter} \times (\text{Shorter Diameter})^2 / 2;$$

and
a relative tumor volume was calculated by the equation:

$$\text{Relative Tumor Volume} = \text{Tumor Volume on Measurement Day} / \text{Tumor Volume on Grouping Day},$$

assuming that the tumor volume on the grouping day was 1. The relative tumor volume was graphed for 13 days after the administration until the tumor of any one of the mice reached a volume exceeding 2,000 $mm^3$. In Fig. 11, it was found that administration of 5 MBq/body of the $^{131}$I-lactosome alone had a weak antitumor effect.

[Reference Example 3: Antitumor Test on Mice by Administration of $^{131}$I-Lactosome (40MBq/body) alone / without PEIT]

Cancer-bearing mice were produced by subcutaneous transplantation of human cancer cells in the following manner.

As animals, 7-week-old BALB/c nu/nu mice (SLC) were used. Each of the mice was anesthetized with Somnopentyl. Human pancreas cancer cells (Suit2) were mixed with a Geltrex matrix gel and subcutaneously transplanted in the right arm of each of the mice at $1 \times 10^6$ cells/0. 04 mL. At the time when the cancer tissue reached a size of 5 mm after growth for 13 days, each of the mice was subjected to an antitumor test.

The above cancer-bearing mice were divided into a control group, a lactosome group, a NaI group, and an $^{131}$I-lactosome group. Each group contained 3 cancer-bearing mice.

In the control group, 0.2 mL/body of normal saline was administered to each of the cancer-bearing mice from its tail vein; in the lactosome group, 0.2 mL/body of a lactosome dispersion liquid was administered to each of the cancer-bearing mice from its tail vein; in the NaI group, 40 MBq/0.2 mL/body of an aqueous $Na^{131}I$ solution was administered to each of the cancer-bearing mice from its tail vein; and in the $^{131}$I-Lactosome group, 40 MBq/0.2 mL/body of an $^{131}$I-lactosome dispersion liquid was administered to each of the cancer-bearing mice from its tail vein. The lactosome administered in the lactosome group contained no $^{131}$I-BzPLLA$_{30}$ and was composed of only PSar$_{70}$-PLLA$_{30}$.

After the administration, the tumor volume and the body weight were measured every 2 or 3 days for 15 days. Changes in the tumor volume are shown in Fig. 12, and changes in the body weight are shown in Fig. 13.

It is to be noted that, in Fig. 12, the size of the tumor was measured with a vernier caliper, the tumor volume was calculated by the equation:

$$\text{Tumor Volume (mm}^3\text{)} = \text{Longer diameter} \times (\text{Shorter Diameter})^2 / 2;$$

and
a relative tumor volume was calculated by the equation:

$$\text{Relative Tumor Volume} = \text{Tumor Volume on Measurement Day} / \text{Tumor Volume on Administration Day},$$

assuming that the tumor volume on the administration day was 1. The relative tumor volume was graphed for 15 days after the administration.

It is to be noted that, in Fig. 13, the change in body weight shows an increase or decrease (g) from the administration day, and a body weight gain was calculated by the equation:

Body Weight Gain = Body Weight on Measurement Day - Body Weight on Administration Day.

A statistical significance test was performed using repeated measures (analysis of variance) in JUMP. As can be seen from Fig. 12, an antitumor effect was observed in the $^{131}$I-Lactosome group as compared to the control group. Further, as a result of repeated measures (analysis of variance) in JUMP, a significant difference was also confirmed. However, as can be seen from Fig. 13, the body weight was reduced by administration of the $^{131}$I-lactosome, and therefore it is considered that radiation produced side effects.

In the above embodiment, the lactosome nanoparticle composed of a linear amphiphilic block polymer has been described as an example. The hydrophilic block of the amphiphilic block polymer may have a branched structure. When the hydrophilic block has a branched structure instead of a linear structure, the hydrophilic shell part of a core/shell structure becomes denser, and therefore a nanoparticle can be formed even when the number of sarcosine units is smaller. Further, a lactosome nanoparticle having a smaller particle diameter can be easily obtained.

## Claims

1. A nanoparticle, comprising:

   an amphiphilic block polymer comprising a hydrophilic block having a sarcosine unit and a hydrophobic block having a lactic acid unit; and a substance labeled with a radioactive emitting nuclide,
   **characterized in that** the radioactive emitting nuclide is a β-ray emitting nuclide, and that the nanoparticle is for internal radiation therapy of a lesion site treated with percutaneous local therapy.

2. The nanoparticle according to claim 1, wherein the β-ray emitting nuclide is selected from the group consisting of iodine-131, yttrium-90, and lutetium-177.

3. The nanoparticle according to claim 1 or 2, wherein the amphiphilic block polymer comprises a hydrophilic block having 20 or more sarcosine units and a hydrophobic block having 10 or more lactic acid units.

4. The nanoparticle according to any one of claims 1 to 3, wherein the nanoparticle has a particle size of 10 nm to 200 nm.

5. The nanoparticle according to any one of claims 1 to 4, wherein the substance labeled with a β-ray emitting nuclide is polylactic acid labeled with a β-ray emitting nuclide.

6. A system for internal radiation therapy of a lesion site comprising:

   a device comprising a means for acquiring image data showing a position of a lesion site, and a means for positioning a needle, which should be punctured into the lesion site, at the lesion site based on the image data; and a nanoparticle according to claim 1.

7. The system according to claim 6, wherein the needle is selected from the group consisting of an injection needle to supply ethanol, an injection needle to supply gas, a radiofrequency electrode needle, and a microwave electrode needle.

8. The system according to claim 6 or 7, wherein the β-ray emitting nuclide is selected from the group consisting of iodine-131, yttrium-90, and lutetium-177.

9. The system according to any one of claims 6 to 8, wherein the amphiphilic block polymer comprises a hydrophilic block having 20 or more sarcosine units and a hydrophobic block having 10 or more lactic acid units.

10. The system according to any one of claims 6 to 9, wherein the nanoparticle has a particle size of 10 nm to 200 nm.

11. The system according to any one of claims 6 to 10, wherein the substance labeled with a β-ray emitting nuclide is polylactic acid labeled with a β-ray emitting nuclide.

**12.** The system according to any one of claims 6 to 11, further comprising a nanoparticle comprising:

an amphiphilic block polymer comprising a hydrophilic block having a sarcosine unit and a hydrophobic block having a lactic acid unit;
and a substance labeled with a γ-ray emitting nuclide.

**13.** The system according to claim 12, wherein the γ-ray emitting nuclide is a single photon emitting nuclide.

**14.** The system according to claim 12, wherein the γ-ray emitting nuclide is a positron emitting nuclide.

**Patentansprüche**

**1.** Nanopartikel, umfassend:

ein amphiphiles Blockpolymer umfassend einen hydrophilen Block, welcher eine Sarkosin-Einheit aufweist, und einen hydrophoben Block, welcher eine Milchsäure-Einheit aufweist, und eine Substanz, die mit einem radioaktiven emittierenden Nuklid markiert ist,
**dadurch gekennzeichnet, dass** das radioaktive emittierende Nuklid ein β-Strahlung emittierendes Nuklid ist, und dass das Nanopartikel zur internen Strahlentherapie einer mit perkutaner lokaler Therapie behandelten Läsionsstelle bestimmt ist.

**2.** Nanopartikel gemäß Anspruch 1, wobei das β-Strahlung emittierende Nuklid aus der Gruppe bestehend aus Iod-131, Yttrium-90 und Lutenium-177 ausgewählt ist.

**3.** Nanopartikel gemäß Anspruch 1 oder 2, wobei das amphiphile Blockpolymer einen hydrophilen Block mit 20 oder mehr Sarkosin-Einheiten und einen hydrophoben Block mit 10 oder mehr Milchsäure-Einheiten umfasst.

**4.** Nanopartikel gemäß irgendeinem der Ansprüche 1 bis 3, wobei das Nanopartikel eine Partikelgröße von 10 nm bis 200 nm aufweist.

**5.** Nanopartikel gemäß irgendeinem der Ansprüche 1 bis 4, wobei die Substanz, die mit einem β-Strahlung emittierenden Nuklid markiert ist, mit einem β-Strahlung emittierenden Nuklid markierte Polymilchsäure ist.

**6.** System für die interne Strahlentherapie einer Läsionsstelle, umfassend:

eine Vorrichtung umfassend Mittel zur Erfassung von Bilddaten, die eine Position einer Läsionsstelle zeigen, und Mittel zur Positionierung einer Nadel, mit der die Läsionsstelle punktiert werden soll, an der Läsionsstelle auf Grundlage der Bilddaten; und
ein Nanopartikel gemäß Anspruch 1.

**7.** System gemäß Anspruch 6, wobei die Nadel aus der Gruppe bestehend aus einer Injektionsnadel zur Abgabe von Ethanol, einer Injektionsnadel zur Abgabe von Gas, einer Radiofrequenz-Elektroden-Nadel und einer Mikrowellen-Elektroden-Nadel ausgewählt ist.

**8.** System gemäß Anspruch 6 oder 7, wobei das β-Strahlung emittierende Nuklid aus der Gruppe bestehend aus Iod-131, Yttrium-90 und Lutenium-177 ausgewählt ist.

**9.** System gemäß irgendeinem der Ansprüche 6 bis 8, wobei das amphiphile Blockpolymer einen hydrophilen Block mit 20 oder mehr Sarkosin-Einheiten und einen hydrophoben Block mit 10 oder mehr Milchsäure-Einheiten umfasst.

**10.** System gemäß irgendeinem der Ansprüche 6 bis 9, wobei das Nanopartikel eine Partikelgröße von 10 nm bis 200 nm aufweist.

**11.** System gemäß irgendeinem der Ansprüche 6 bis 10, wobei die Substanz, die mit einem β-Strahlung emittierenden Nuklid markiert ist, mit einem β-Strahlung emittierenden Nuklid markierte Polymilchsäure ist.

**12.** System gemäß irgendeinem der Ansprüche 6 bis 11, ferner umfassend ein Nanopartikel, welches umfasst:

ein amphiphiles Blockpolymer umfassend einen hydrophilen Block, welcher eine Sarkosin-Einheit aufweist, und einen hydrophoben Block, welcher eine Milchsäure-Einheit aufweist;
und eine Substanz, die mit einem γ-Strahlung emittierenden Nuklid markiert ist.

**13.** System gemäß Anspruch 12, wobei das γ-Strahlung emittierende Nuklid ein Einzelphotonen emittierendes Nuklid ist.

**14.** System gemäß Anspruch 12, wobei das γ-Strahlung emittierende Nuklid ein Positronen emittierendes Nuklid ist.

## Revendications

**1.** Nanoparticule, comprenant :

un polymère bloc amphiphile comprenant un bloc hydrophile comportant une unité de sarcosine et un bloc hydrophobe comportant une unité d'acide lactique ; et une substance marquée avec un nucléide émetteur de radioactivité,
**caractérisée en ce que** le nucléide émetteur de radioactivité est un nucléide émetteur de rayonnement β, et **en ce que** la nanoparticule est destinée à une radiothérapie interne d'un site de lésion traité par un traitement local percutané.

**2.** Nanoparticule selon la revendication 1, dans laquelle le nucléide émetteur de rayonnement β est sélectionné dans le groupe consistant en iode-131, yttrium-90, et lutécium-177.

**3.** Nanoparticule selon la revendication 1 ou 2, dans laquelle le polymère bloc amphiphile comprend un bloc hydrophile comportant 20 unités de sarcosine ou plus et un bloc hydrophobe comportant 10 unités d'acide lactique ou plus.

**4.** Nanoparticule selon l'une quelconque des revendications 1 à 3, dans laquelle la nanoparticule a une taille de particule de 10 nm à 200 nm.

**5.** Nanoparticule selon l'une quelconque des revendications 1 à 4, dans laquelle la substance marquée avec un nucléide émetteur de rayonnement β est un acide polylactique marqué avec un nucléide émetteur de rayonnement β.

**6.** Système pour radiothérapie interne d'un site de lésion comprenant :

un dispositif comprenant un moyen pour acquérir des données d'images montrant une position d'un site de lésion, et un moyen pour positionner une aiguille, qui devra perforer le site de lésion, au site de lésion en se basant sur les données d'images ; et
une nanoparticule selon la revendication 1.

**7.** Système selon la revendication 6, dans lequel l'aiguille est sélectionnée dans le groupe consistant en une aiguille d'injection pour fournir l'éthanol, une aiguille d'injection pour fournir le gaz, une aiguille d'électrode à radiofréquence, et une aiguille d'électrode à micro-onde.

**8.** Système selon la revendication 6 ou 7, dans lequel le nucléide émetteur de rayonnement β est sélectionné dans le groupe consistant en iode-131, yttrium-90, et lutécium-177.

**9.** Système selon l'une quelconque des revendications 6 à 8, dans lequel le polymère bloc amphiphile comprend un bloc hydrophile comportant 20 unités de sarcosine ou plus et un bloc hydrophobe comportant 10 unités d'acide lactique ou plus.

**10.** Système selon l'une quelconque des revendications 6 à 9, dans lequel la nanoparticule a une taille de particule de 10 nm à 200 nm.

**11.** Système selon l'une quelconque des revendications 6 à 10, dans lequel la substance marquée avec un nucléide émetteur de rayonnement β est un acide polylactique marqué avec un nucléide émetteur de rayonnement β.

**12.** Système selon l'une quelconque des revendications 6 à 11, comprenant en outre une nanoparticule comprenant :

un polymère bloc amphiphile comprenant un bloc hydrophile comportant une unité de sarcosine et un bloc hydrophobe comportant une unité d'acide lactique ;
et une substance marquée avec un nucléide émetteur de rayonnement $\gamma$.

**13.** Système selon la revendication 12, dans lequel le nucléide émetteur de rayonnement $\gamma$ est un nucléide émetteur de photon unique.

**14.** Système selon la revendication 12, dans lequel le nucléide émetteur de rayonnement $\gamma$ est un nucléide émetteur de positron.

Fig.1

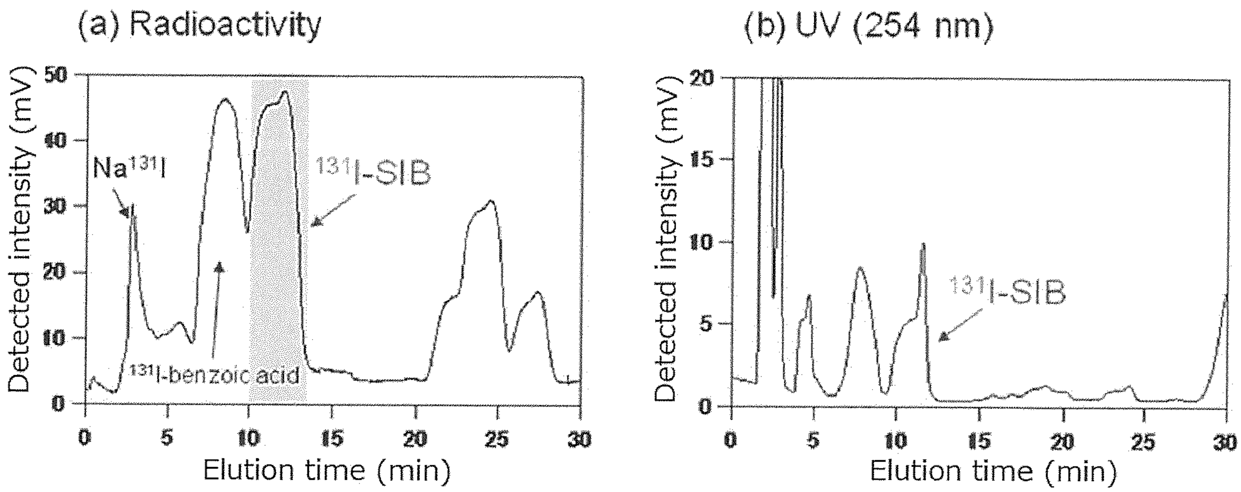
(a) Radioactivity
Detected intensity (mV)
Na131I
131I-SIB
131I-benzoic acid
Elution time (min)
(b) UV (254 nm)
Detected intensity (mV)
131I-SIB
Elution time (min)

Fig.2

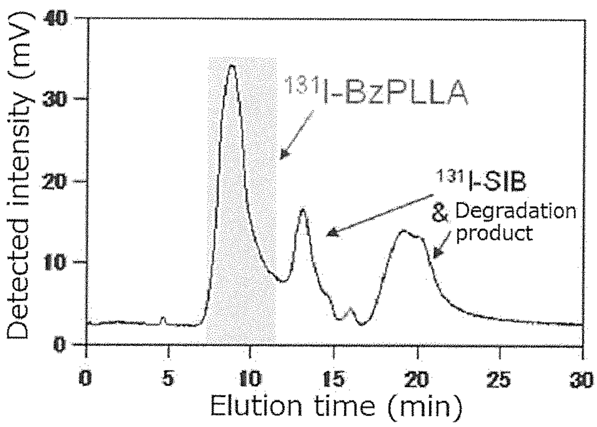
Detected intensity (mV)
131I-BzPLLA
131I-SIB
& Degradation product
Elution time (min)

Fig.3

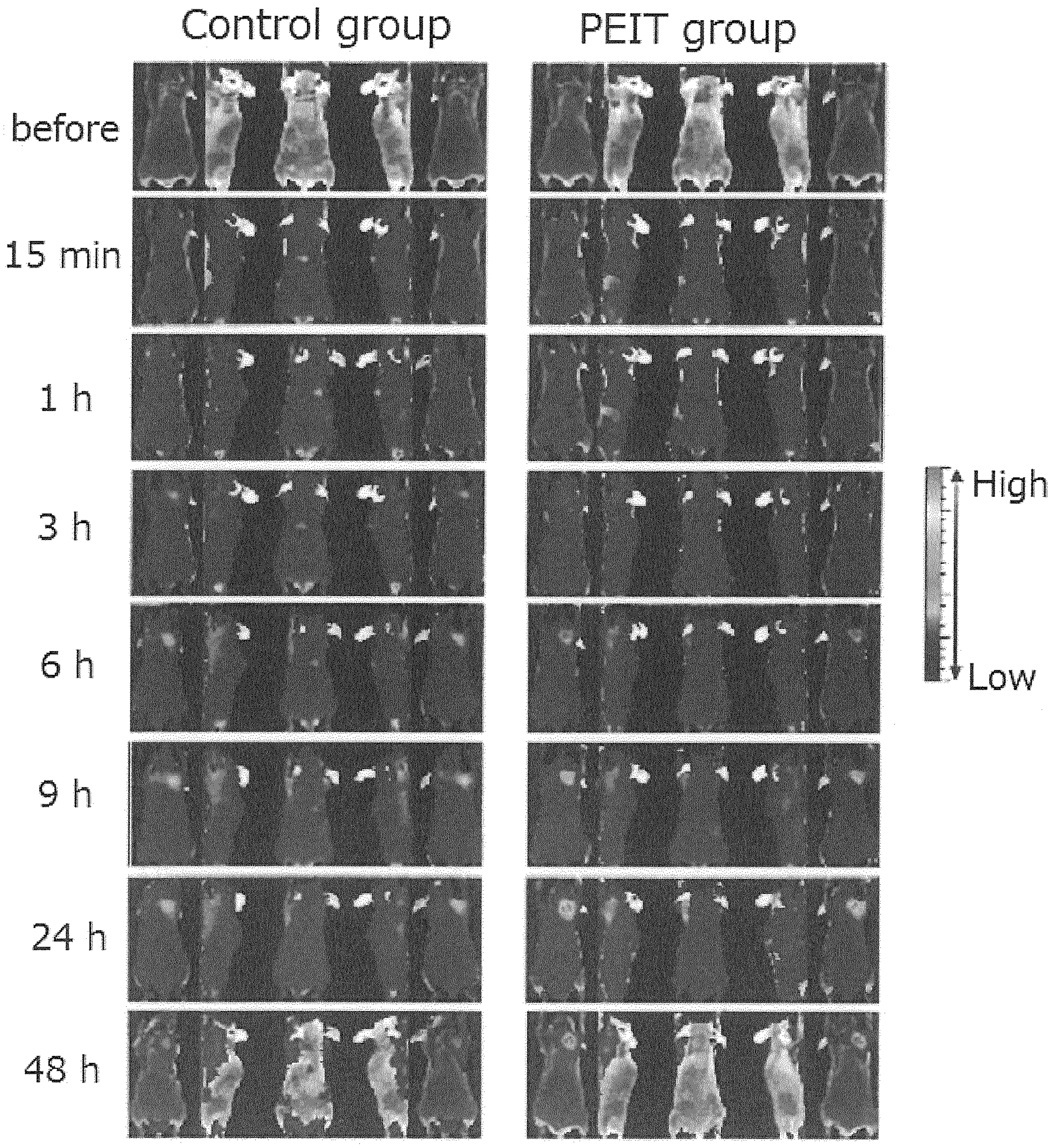
Control group
PEIT group
before
15 min
1 h
3 h
6 h
9 h
24 h
48 h
High
Low

Fig.4

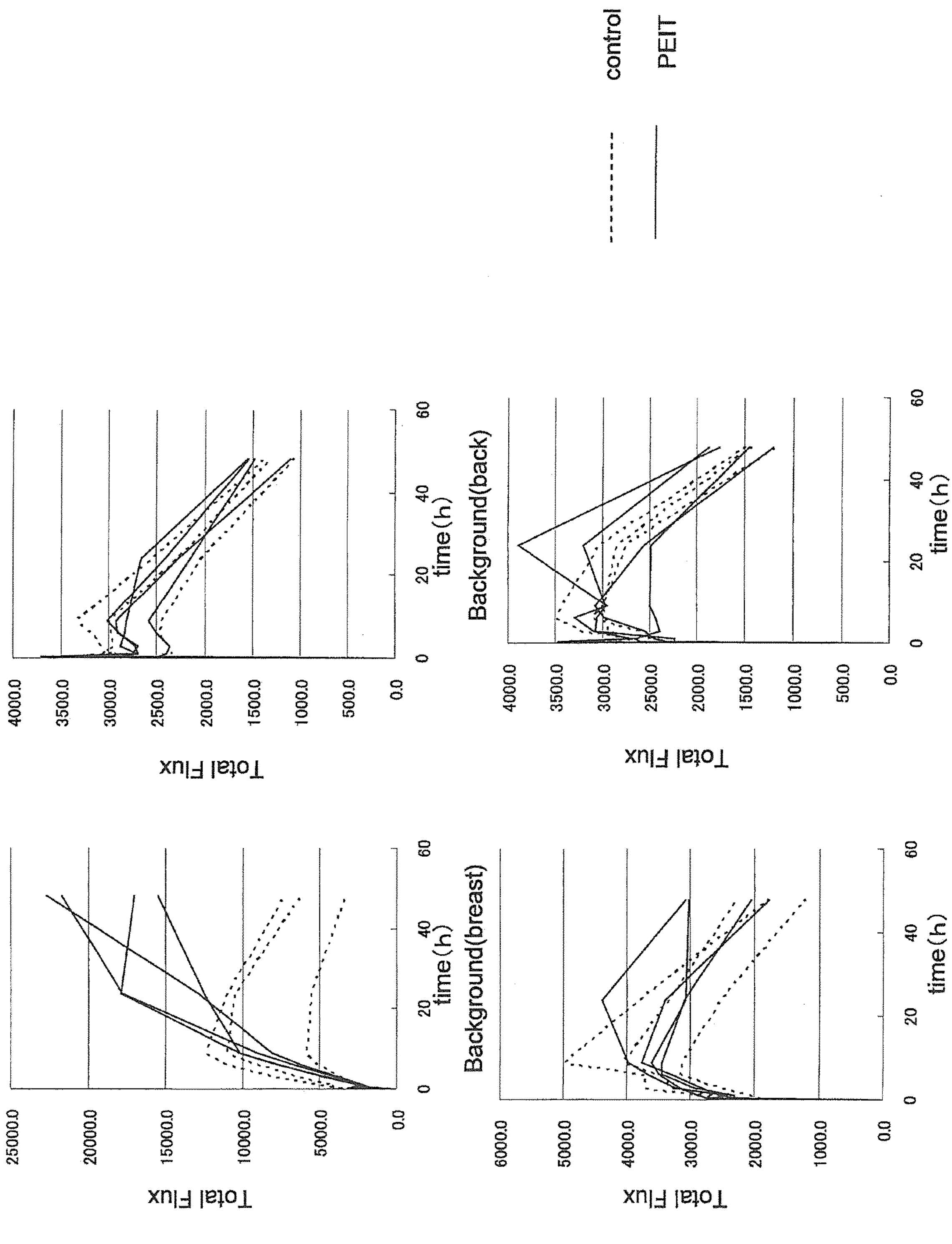
tumor
liver
Background(breast)
Background(back)
Total Flux
time (h)
0
20
40
60
25000.0
20000.0
15000.0
10000.0
5000.0
0.0
4000.0
3500.0
3000.0
2500.0
2000.0
1500.0
1000.0
500.0
6000.0
5000.0
control
PEIT

Fig.5

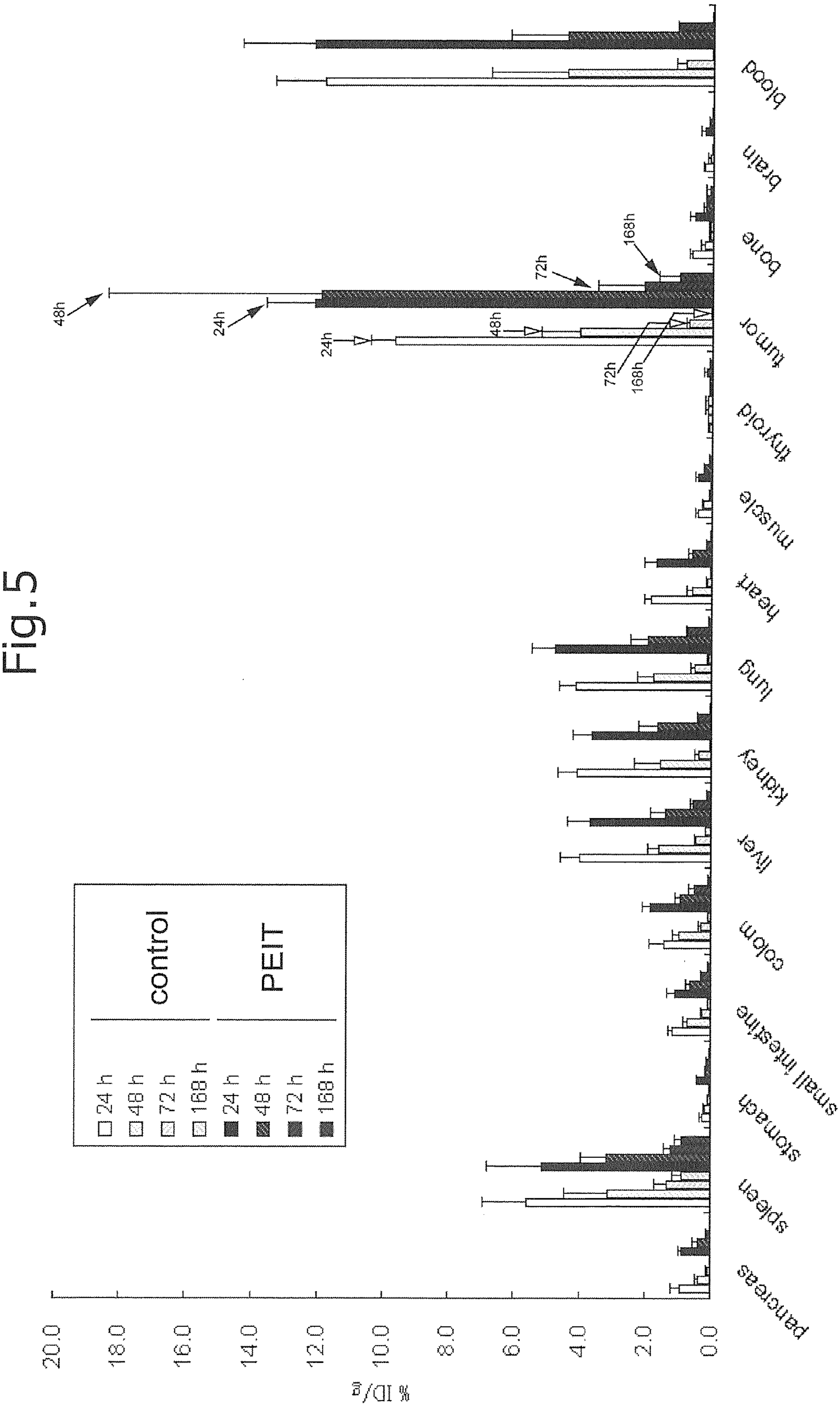

24 h
48 h
72 h
168 h
control
24 h
48 h
72 h
168 h
PEIT
48h
24h
72h
168h
24h
48h
72h
168h
20.0
18.0
16.0
14.0
12.0
10.0
8.0
6.0
4.0
2.0
0.0
% ID/g
pancreas
spleen
stomach
small intestine
colom
liver
kidney
lung
heart
muscle
thyroid
tumor
bone
brain
blood

Fig.6

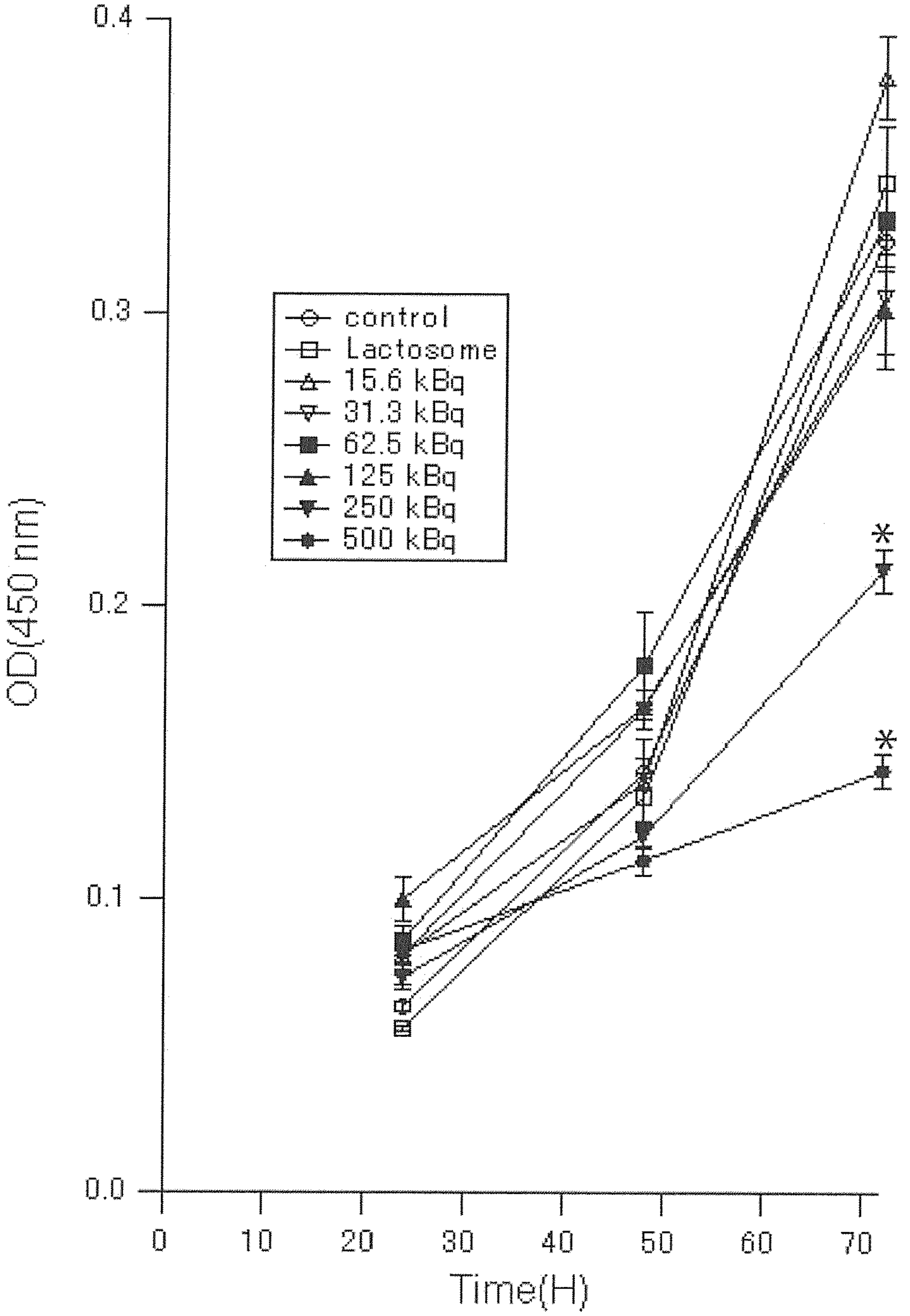
0.4
0.3
0.2
0.1
0.0
OD(450 nm)
control
Lactosome
15.6 kBq
31.3 kBq
62.5 kBq
125 kBq
250 kBq
500 kBq
*
*
0
10
20
30
40
50
60
70
Time(H)

Fig.7

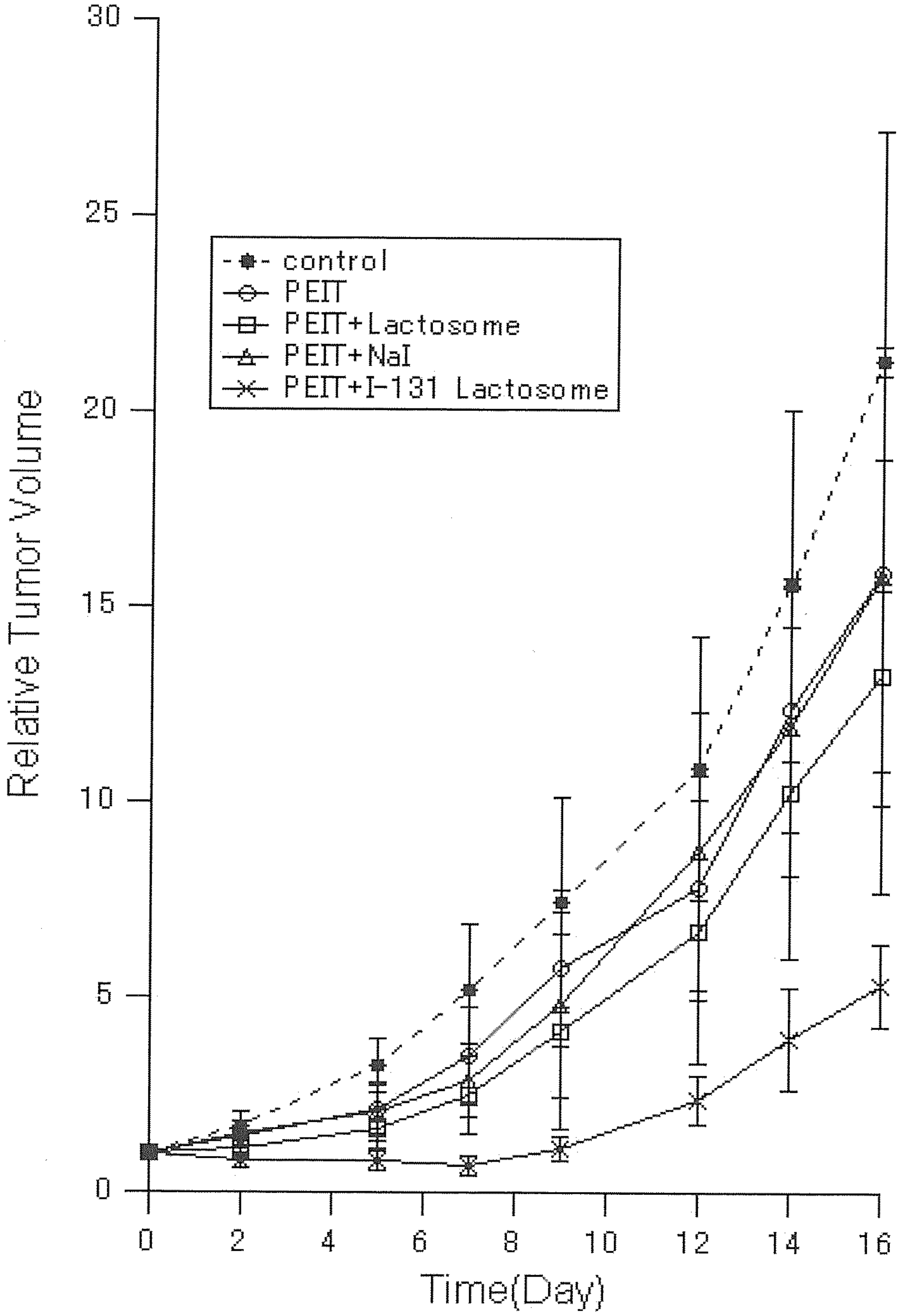
30
25
20
15
10
5
0
Relative Tumor Volume
0
2
4
6
8
10
12
14
16
Time(Day)
control
PEIT
PEIT+Lactosome
PEIT+NaI
PEIT+I-131 Lactosome

Fig.8

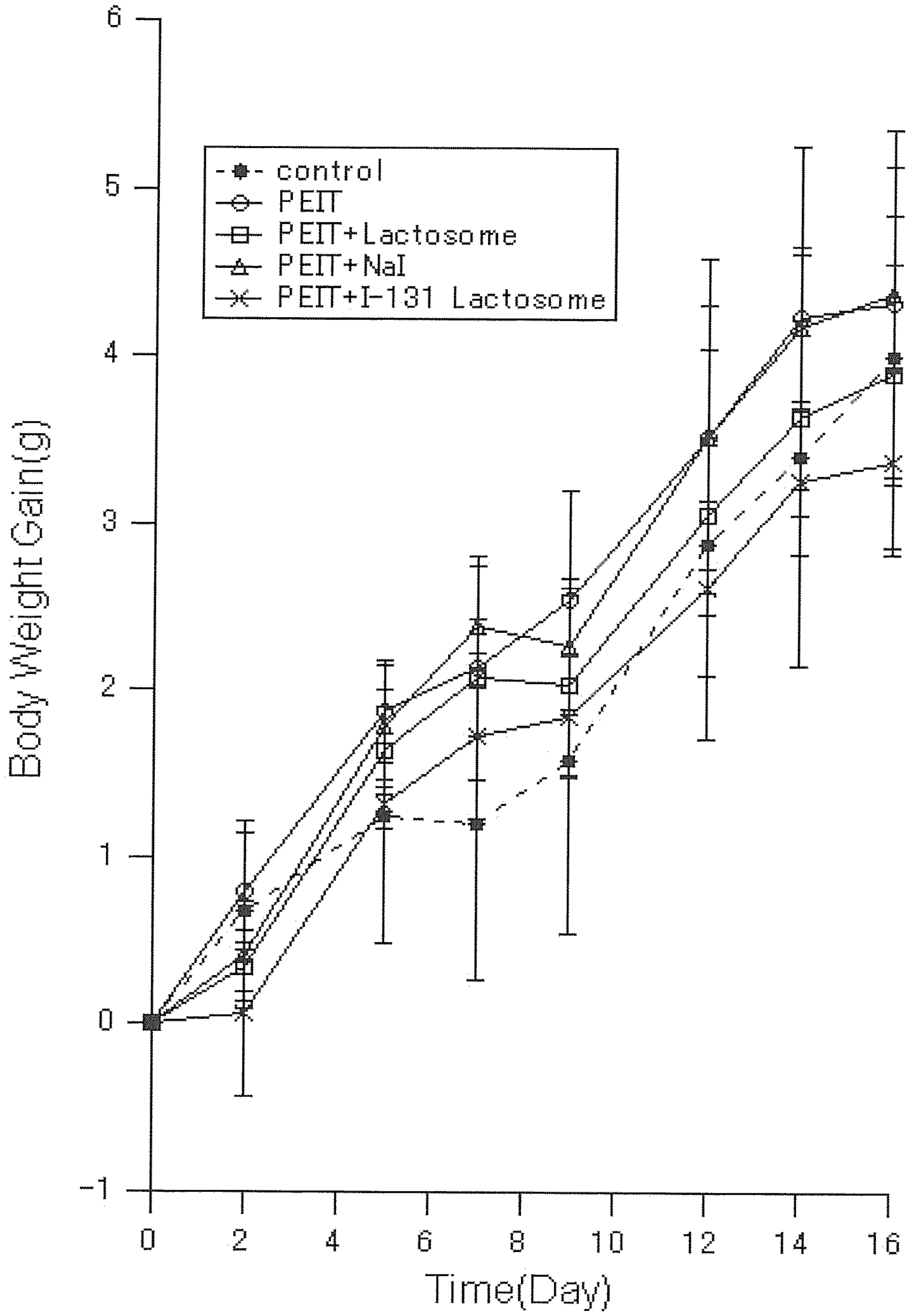
control
PEIT
PEIT+Lactosome
PEIT+NaI
PEIT+I-131 Lactosome
Body Weight Gain(g)
Time(Day)
6
5
4
3
2
1
0
-1
0
2
4
6
8
10
12
14
16

Fig.9

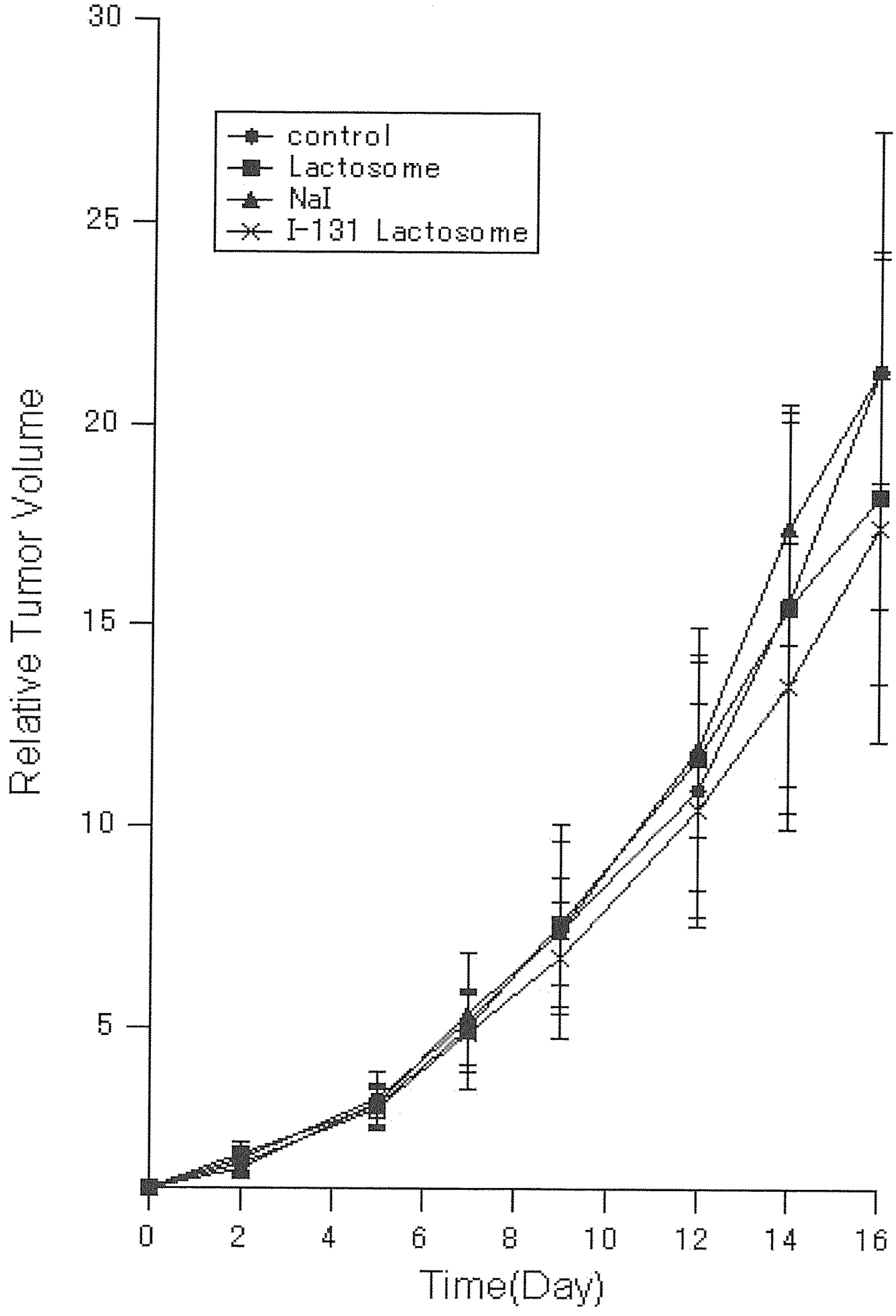

30
25
20
15
10
5
Relative Tumor Volume
control
Lactosome
NaI
I-131 Lactosome
0
2
4
6
8
10
12
14
16
Time(Day)

Fig.10

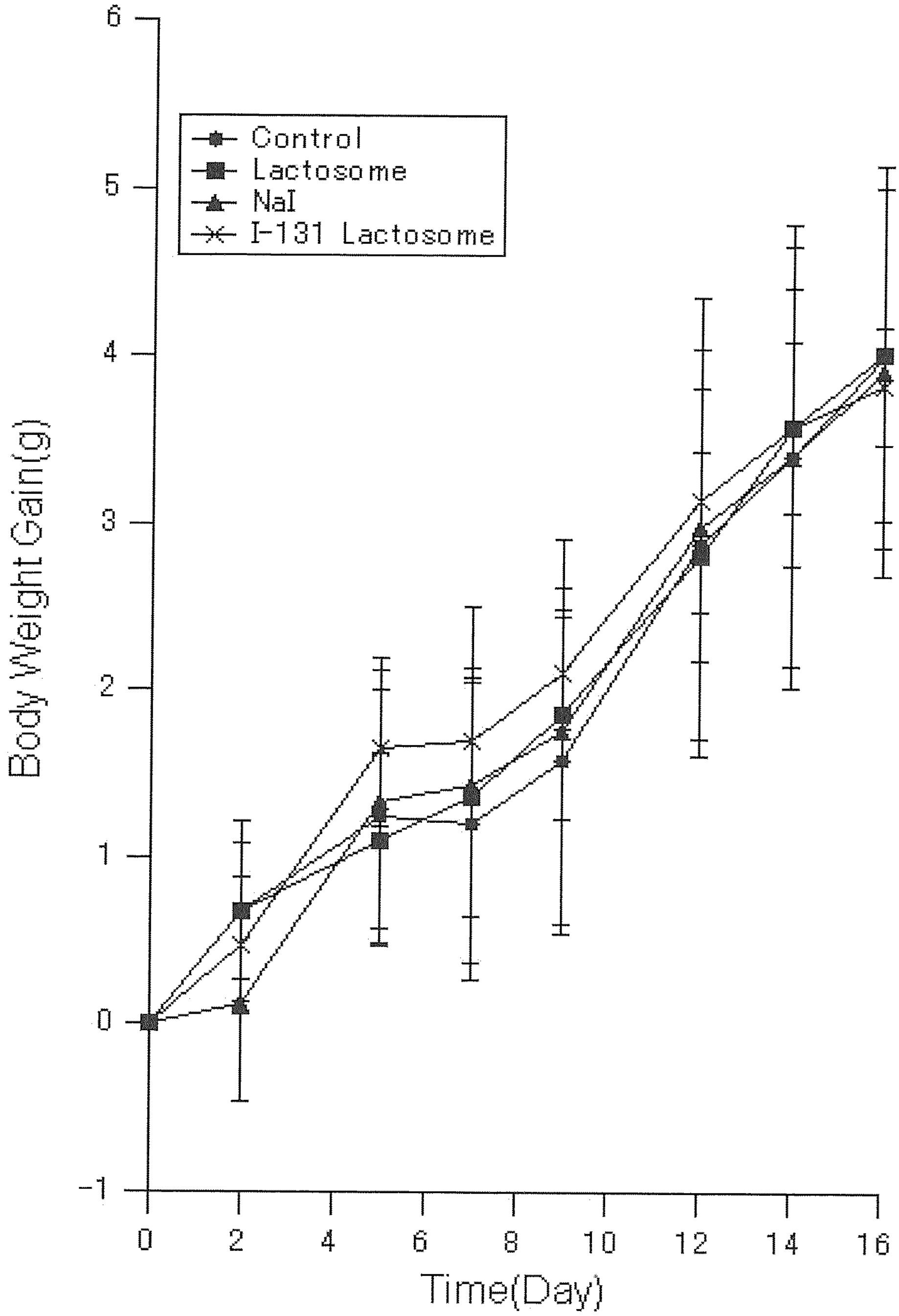
Control
Lactosome
NaI
I-131 Lactosome
Body Weight Gain(g)
Time(Day)
6
5
4
3
2
1
0
-1
0
2
4
6
8
10
12
14
16

Fig.11

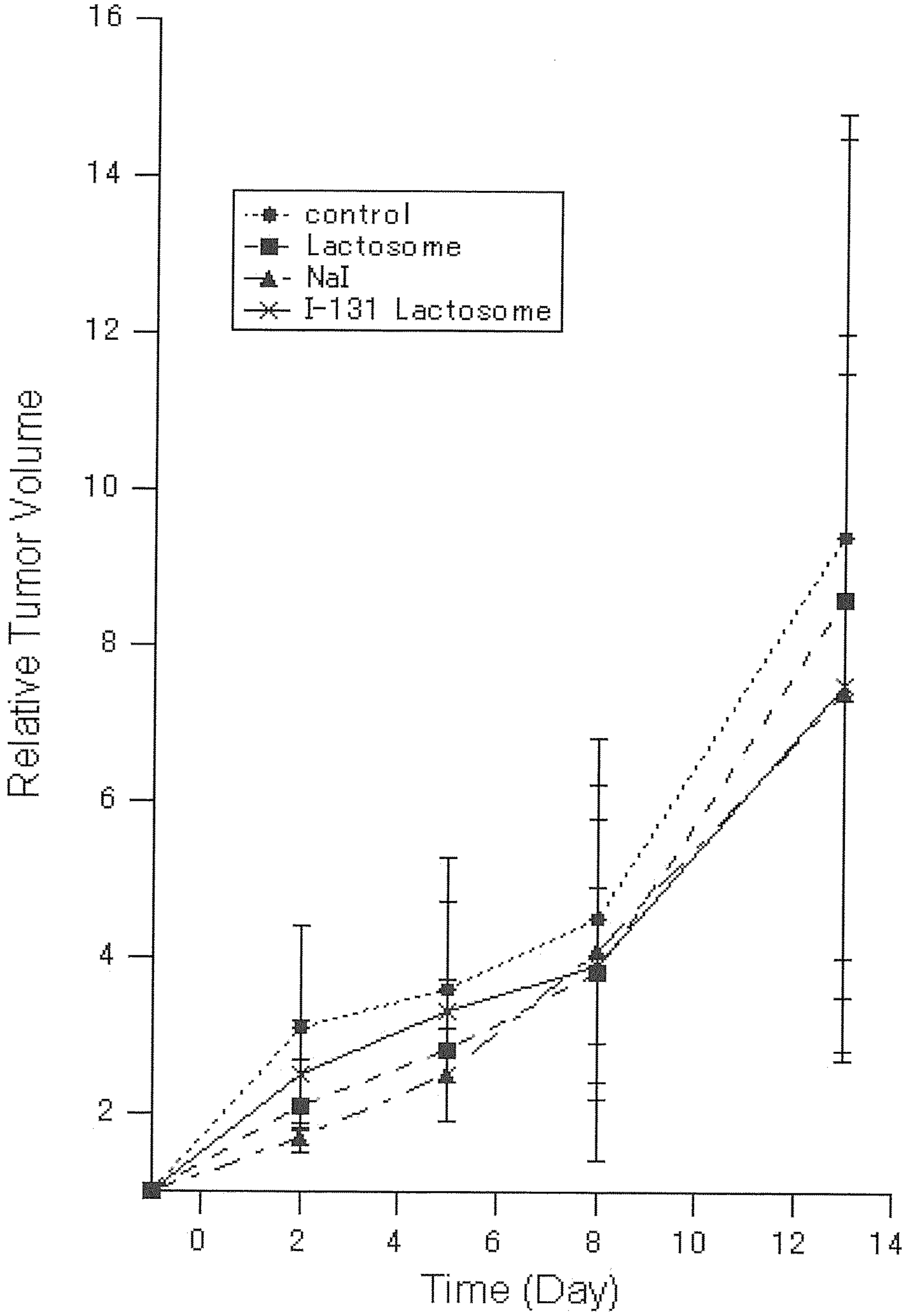
control
Lactosome
NaI
I-131 Lactosome
Relative Tumor Volume
Time (Day)
0
2
4
6
8
10
12
14
16

Fig.12

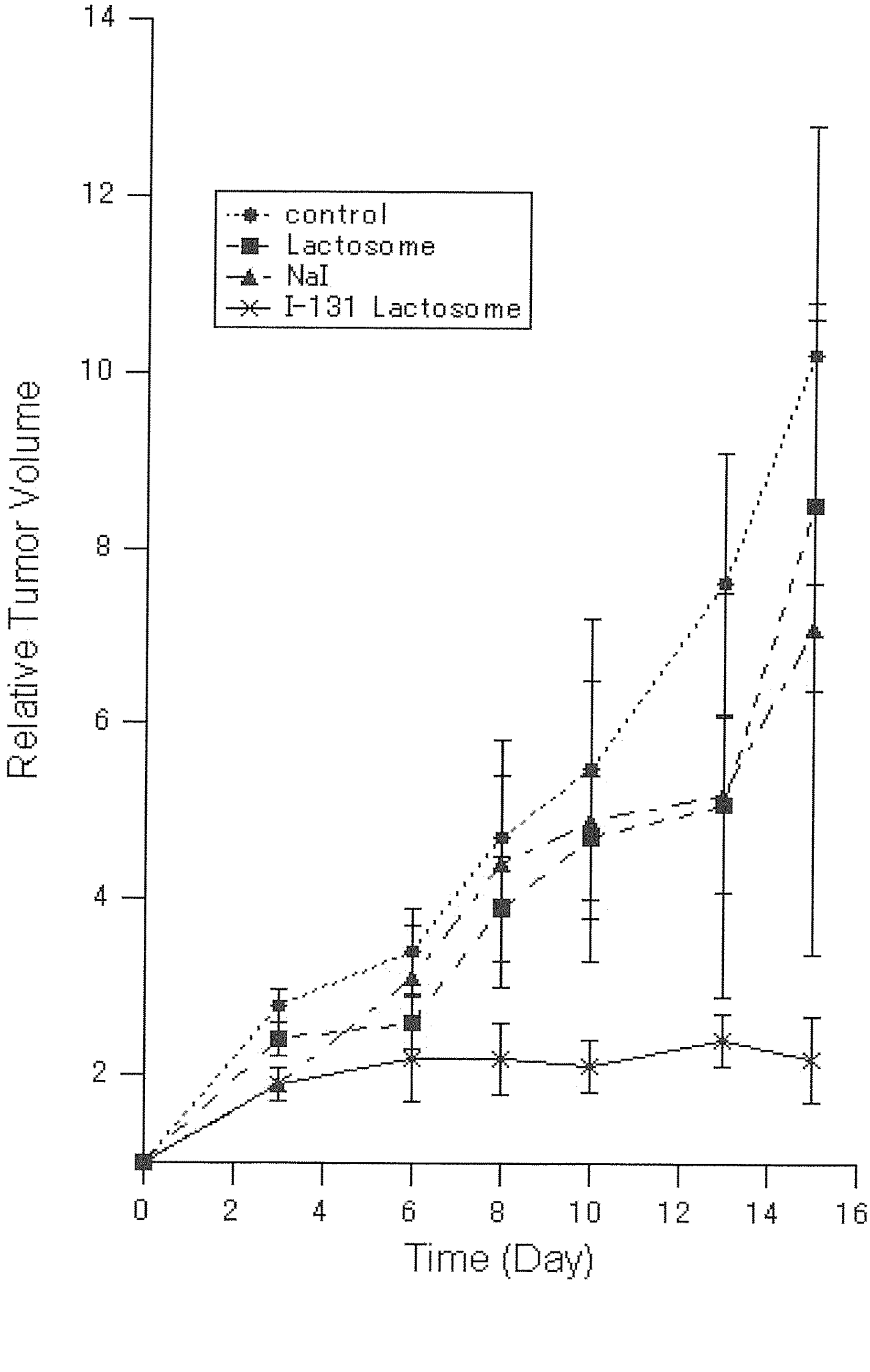
control
Lactosome
NaI
I-131 Lactosome
Relative Tumor Volume
Time (Day)
0
2
4
6
8
10
12
14
16

Fig.13

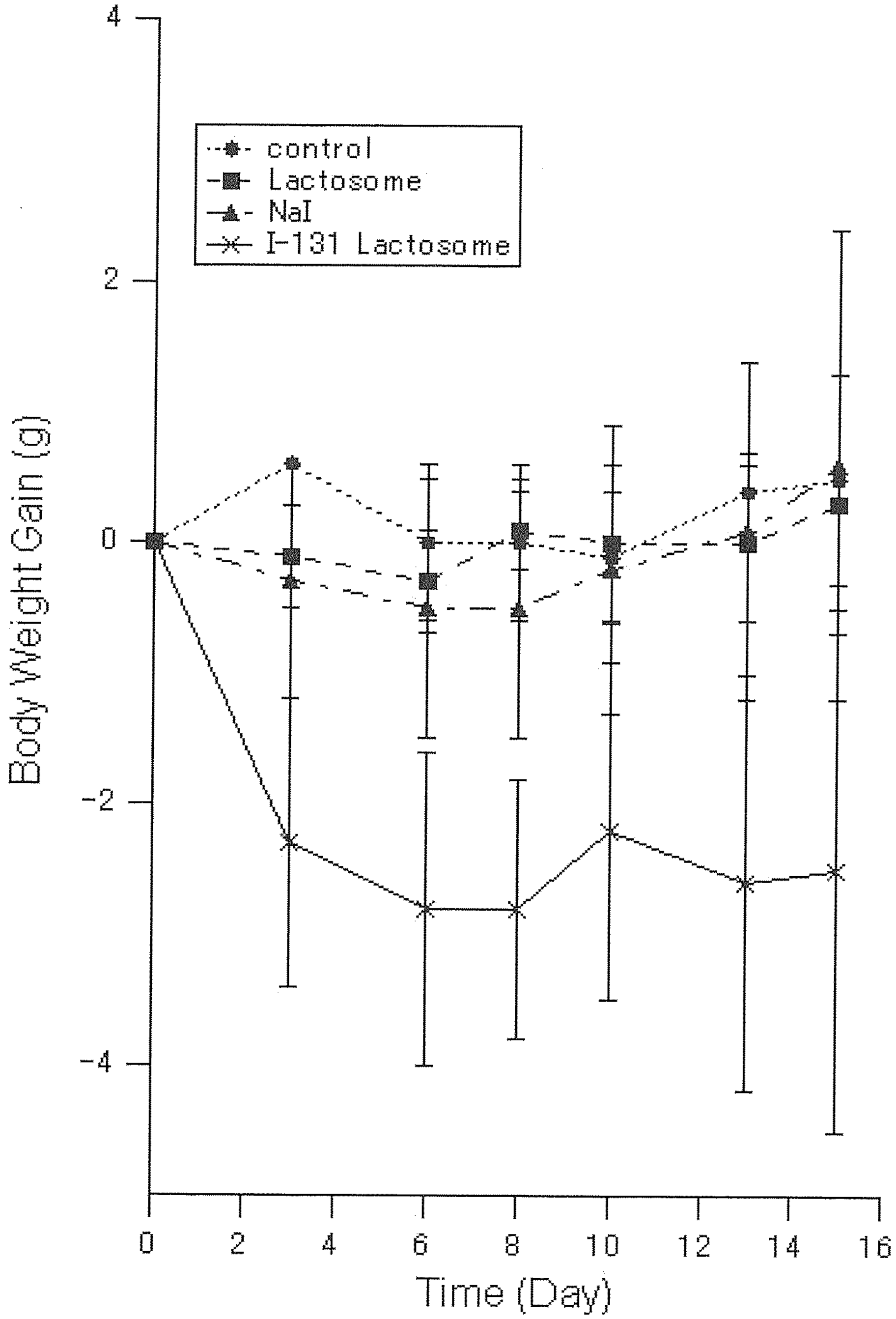
control
Lactosome
NaI
I-131 Lactosome
Body Weight Gain (g)
Time (Day)
4
2
0
-2
-4
0
2
4
6
8
10
12
14
16

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2009148121 A

### Non-patent literature cited in the description

- *Abstract of The 50th Annual Scientific Meeting of the Japanese Society of Nuclear Medicine,* 2010, 316-321